# EUROPEAN PATENT SPECIFICATION

(11) **EP 2 524 033 B1**
(45) Date of publication and mention of the grant of the patent: **13.04.2016**
(21) Application number: 11733018.3
(22) Date of filing: 17.01.2011
(51) Int. Cl.: C12Q 1/68

(54) **METHOD FOR SCREENING INDUCED PLURIPOTENT STEM CELLS**
VERFAHREN ZUM SCREENING INDUZIERTER PLURIPOTENTER STAMMZELLEN
PROCÉDÉ DE CRIBLAGE DE CELLULES SOUCHES PLURIPOTENTES INDUITES

(30) Priority: 15.01.2010 US 282295 P
(43) Date of publication of application: 21.11.2012
(73) Proprietor: Kyoto University, Kyoto-shi, Kyoto 606-8501 (JP)
(72) Inventor: YAMANAKA, Shinya, Kyoto-shi Kyoto 606-8507 (JP); KOYANAGI, Michiyo, Kyoto-shi Kyoto 606-8507 (JP)
(74) Representative: D Young & Co LLP
(86) International application number: PCT/JP2011/051144
(87) International publication number: WO 2011/087154

(56) References cited:
- MARJORIE PICK ET AL: "Clone- and Gene-Specific Aberrations of Parental Imprinting in Human Induced Pluripotent Stem Cells", STEM CELLS, vol. 27, no. 11, 1 November 2009 (2009-11-01), pages 2686-2690, XP055062998, ISSN: 1066-5099, DOI: 10.1002/stem.205
- MARK H. CHIN ET AL: "Induced Pluripotent Stem Cells and Embryonic Stem Cells Are Distinguished by Gene Expression Signatures", CELL STEM CELL, vol. 5, no. 1, 1 July 2009 (2009-07-01), pages 111-123, XP055013700, ISSN: 1934-5909, DOI: 10.1016/j.stem.2009.06.008
- Anonymous: "GeneChip miRNA Array", , 1 January 2009 (2009-01-01), pages 1-2, XP055035969, Retrieved from the Internet: URL:http://media.affymetrix.com/support/te chnical/datasheets/miRNA_datasheet.pdf [retrieved on 2012-08-21]
- SHAU-PING LIN ET AL: "Asymmetric regulation of imprinting on the maternal and paternal chromosomes at the Dlk1-Gtl2 imprinted cluster on mouse chromosome 12", NATURE GENETICS, vol. 35, no. 1, 1 September 2003 (2003-09-01), pages 97-102, XP055063225, ISSN: 1061-4036, DOI: 10.1038/ng1233
- JOHN P HAGAN ET AL: "At Least Ten Genes Define the Imprinted Dlk1-Dio3 Cluster on Mouse Chromosome 12qF1", PLOS ONE, PUBLIC LIBRARY OF SCIENCE, US, vol. 4, no. 2, 1 March 2009 (2009-03-01), pages 1-19, XP008159400, ISSN: 1932-6203, DOI: 10.1371/JOURNAL.PONE.0004352 [retrieved on 2009-02-05]
- KITCHENER D. WILSON ET AL: "MicroRNA Profiling of Human-Induced Pluripotent Stem Cells", STEM CELLS AND DEVELOPMENT, vol. 18, no. 5, 1 June 2009 (2009-06-01), pages 749-757, XP055063091, ISSN: 1547-3287, DOI: 10.1089/scd.2008.0247
- KEISUKE OKITA ET AL: "Generation of germline-competent induced pluripotent stem cells", NATURE: INTERNATIONAL WEEKLY JOURNAL OF SCIENCE, NATURE PUBLISHING GROUP, UNITED KINGDOM, vol. 448, 1 July 2007 (2007-07-01), pages 313-317, XP008137105, ISSN: 0028-0836
- L. LIU ET AL: "Activation of the Imprinted Dlk1-Dio3 Region Correlates with Pluripotency Levels of Mouse Stem Cells", JOURNAL OF BIOLOGICAL CHEMISTRY, vol. 285, no. 25, 18 June 2010 (2010-06-18), pages 19483-19490, XP055063001, ISSN: 0021-9258, DOI: 10.1074/jbc.M110.131995
- MATTHIAS STADTFELD ET AL: "Aberrant silencing of imprinted genes on chromosome 12qF1 in mouse induced pluripotent stem cells", NATURE, vol. 465, no. 7295, 13 May 2010 (2010-05-13), pages 175-181, XP055062999, ISSN: 0028-0836, DOI: 10.1038/nature09017
- ERNESTO LUJAN ET AL: "An imprinted signature helps isolate ESC-equivalent iPSCs", CELL RESEARCH, vol. 20, no. 9, 10 August 2010 (2010-08-10), pages 974-976, XP055063089, ISSN: 1001-0602, DOI: 10.1038/cr.2010.117
- PICK,M. ET AL.: 'Clone- and gene-specific aberrations of parental imprinting in human induced pluripotent stem cells.' STEM CELLS vol. 27, no. 11, November 2009, pages 2686 - 2690, XP055062998
- CHIN,M.H. ET AL.: 'Induced pluripotent stem cells and embryonic stem cells are distinguished by gene expression signatures.' CELL STEM CELL vol. 5, no. 1, 02 July 2009, pages 111 - 123, XP055013700
- STADTFELD,M. ET AL.: 'Aberrant silencing of imprinted genes on chromosome 12qFl in mouse induced pluripotent stem cells.' NATURE vol. 465, no. 7295, 13 May 2010, pages 175 - 181, XP055062999
- LIU,L. ET AL.: 'Activation of the imprinted Dlkl-Dio3 region correlates with pluripotency levels of mouse stem cells.' J. BIOL. CHEM. vol. 285, no. 25, 18 June 2010, pages 19483 - 19490, XP055063001
- OKITA,K. ET AL.: 'Generation of germline-competent induced pluripotent stem cells.' NATURE vol. 448, no. 7151, 19 July 2007, pages 313 - 317, XP002555950
- HAGAN, J. P. ET AL.: 'At least ten genes define the imprinted Dlkl-Dio3 cluster on mouse chromosome 12qFl.' PLOS ONE vol. 4, no. 2, E4352, 05 February 2009, pages 1 - 19, XP008159400
- KAZUTOSHI TAKAHASHI ET AL: "Induction of Pluripotent Stem Cells from Mouse Embryonic and Adult Fibroblast Cultures by Defined Factors", CELL, CELL PRESS, US, vol. 126, no. 4, 25 August 2006 (2006-08-25), pages 663-676, XP008157317, ISSN: 0092-8674, DOI: 10.1016/J.CELL.2006.07.024
- KAZUTOSHI TAKAHASHI ET AL: "Induction of Pluripotent Stem Cells from Adult Human Fibroblasts by Defined Factors", CELL, CELL PRESS, US, vol. 131, no. 5, 30 November 2007 (2007-11-30), pages 861-872, XP008155962, ISSN: 0092-8674, DOI: 10.1016/J.CELL.2007.11.019

<110> Method For Screening Induced Pluripotent Stem Cells
<120> Kyoto University
<130> PH-4105-PCT
<150> US61/282,295
   <151> 2010-01-15
<160> 275
<170> Patent In version 3.4
<210> 1
   <211> 94
   <212> RNA
   <213> Mus musculus
<400> 1
   gccaccuucu gugcccccag caccacgugu cugggccacg ugagcaacgc cacgugggcc 60
   ugacguggag cuggggccgc aggggucuga uggc 94
<210> 2
   <211> 91
   <212> RNA
   <213> Mus musculus
<400> 2
   uggagccuga ggggcucaca gcucuggucc uuggagcucc agagaaaaug uugcuccggg 60
   gcugaguucu gugcaccccc cuugcccucc a 91
<210> 3
   <211> 83
   <212> RNA
   <213> Mus musculus
<400> 3
   cgccagggcc uuguacaugg uaggcuuuca uucauuuuuu gcacauucgg ugaagguccu 60
   acugugugcc aggcccugug cca 83
<210> 4
   <211> 97
   <212> RNA
   <213> Mus musculus
<400> 4
   caguguagug agaaguuggg gggugggaac ggcgucaugc aggaguugau ugcacagcca 60
   uucagcuccu auaugaugcc uuucuucacc cccuuca 97
<210> 5
   <211> 67
   <212> RNA
   <213> Mus musculus
<400> 5
   ugggccaagg gucacccucu gacucugugg ccaaggguag acaggucaga ggucgauccu 60
   gggccua 67
<210> 6
   <211> 94
   <212> RNA
   <213> Mus musculus
<400> 6
   agaacagggu cuccuugagg ggccucugcc ucuauccagg auuauguuuu uaugaccagg 60
   aggcugaggu cccuuacagg cggccucuua cucu 94
<210> 7
   <211> 91
   <212> RNA
   <213> Mus musculus
<400> 7
   cguccugcga ggugucuugc aggccgucau gcaggccaca cugacgguaa cguugcaggu 60
   cgucuugcag ggcuucucgc aagacgacau c 91
<210> 8
   <211> 124
   <212> RNA
   <213> Mus musculus
<400> 8
   ugcccgggga gaaguacggu gagccuguca uuauucagag aggcuagauc cucuguguug 60
   agaaggauca ugaugggcuc cucgguguuc uccagguagc ggcaccacac caugaaggca 120
   gccc 124
<210> 9
   <211> 70
   <212> RNA
   <213> Mus musculus
<400> 9
   ccagccugcu gaagcucaga gggcucugau ucagaaagau caucggaucc gucugagcuu 60
   ggcuggucgg 70
<210> 10
   <211> 94
   <212> RNA
   <213> Mus musculus
<400> 10
   ucgacucugg guuugaacca aagcucgacu caugguuuga accauuacuu aauucguggu 60
   uugaaccauc acucgacucc ugguucgaac cauc 94
<210> 11
   <211> 75
   <212> RNA
   <213> Mus musculus
<400> 11
   uggguagcuc uugcauuucc uggugggggc cacuggaugg cuccuccacu ucuuggagua 60
   gaucaguggg cagcu 75
<210> 12
   <211> 62
   <212> RNA
   <213> Mus musculus
<400> 12
   gaggacucca uuuguuuuga ugauggauuc uuaagcucca ucaucgucuc aaaugagucu 60
   uc 62
<210> 13
   <211> 96
   <212> RNA
   <213> Mus musculus
<400> 13
   aaaaugauga ugucaguugg ccggucggcc gaucgcucgg ucugucaguc agucggucgg 60
   ucgaucgguc ggucggucag ucggcuuccu gucuuc 96
<210> 14
   <211> 120
   <212> RNA
   <213> Mus musculus
<400> 14
   auacucacag ucucccagcu ggugugaggu ugggccagga ugaaacccaa ggcucuccga 60
   ggcuccccac cacacccugc ugcugaagac ugccuagcaa ggcugugccg aguggugugg 120
<210> 15
   <211> 79
   <212> RNA
   <213> Mus musculus
<400> 15
   agacggagag accaggucac gucucugcag uuacacagcu caugagugcc ugcuggggug 60
   gaaccugguu ugucugucu 79
<210> 16
   <211> 77
   <212> RNA
   <213> Mus musculus
<400> 16
   cagcaguacc aggagagagu uagcgcauua gugcaauagu uaguccugau uucuggguuu 60
   uucuaauggc ugcucuu 77
<210> 17
   <211> 66
   <212> RNA
   <213> Mus musculus
<400> 17
   agagauggua gacuauggaa cguaggcguu auguuuuuga ccuauguaac augguccacu 60
   aacucu 66
<210> 18
   <211> 82
   <212> RNA
   <213> Mus musculus
<400> 18
   ugguacuugg agagauagua gaccguauag cguacgcuuu aucugugacg uauguaacac 60
   gguccacuaa cccucaguau ca 82
<210> 19
   <211> 63
   <212> RNA
   <213> Mus musculus
<400> 19
   aagaaauggu uuaccguccc acauacauuu ugaguaugua ugugggacgg uaaaccgcuu 60
   cuu 63
<210> 20
   <211> 61
   <212> RNA
   <213> Mus musculus
<400> 20
   aagaugguug accauagaac augcgcuacu ucugugucgu auguaguaug guccacaucu 60
   u 61
<210> 21
   <211> 120
   <212> RNA
   <213> Mus musculus
<400> 21
   gugagcugga aucagccagc guuaccucaa gguauuugaa gaugcgguug accauggugu 60
   guacgcuuua uuuaugacgu aggacacaug gucuacuucu ucucaauauc acaucucgcc 120
<210> 22
   <211> 86
   <212> RNA
   <213> Mus musculus
<400> 22
   uugguacuug gagagaggug guccguggcg cguucgcuuc auuuauggcg cacauuacac 60
   ggucgaccuc uuugcgguau cuaauc 86
<210> 23
   <211> 81
   <212> RNA
   <213> Mus musculus
<400> 23
   ugggugcgug aggugguuga ccagagagca cacgcuauau uugugccguu ugugaccugg 60
   uccacuaacc cucaguaucu a 81
<210> 24
   <211> 97
   <212> RNA
   <213> Mus musculus
<400> 24
   uguucgcuuc ugguaccgga agagagguuu ucugggucuc uguuucuuug augagaauga 60
   aacacaccca gcuaaccuuu uuuucaguau caaaucc 97
<210> 25
   <211> 85
   <212> RNA
   <213> Mus musculus
<400> 25
   uugauacuug aaggagaggu uguccguguu gucuucucuu uauuuaugau gaaacauaca 60
   cgggaaaccu cuuuuuuagu aucaa 85
<210> 26
   <211> 74
   <212> RNA
   <213> Mus musculus
<400> 26
   cuauggcuuu ggacugugag gugacucuug gugugugaug gcuuuucagc aagguccucc 60
   ucacaguagc uaua 74
<210> 27
   <211> 121
   <212> RNA
   <213> Mus musculus
<400> 27
   cugaagggac aaugaugccc acuguucucg ggguagcugu guggauggua gaccggugac 60
   guacacuuca uuuaugcugu aggucacccg uuuuacuauc caccaacacc cagaccaucu 120
   g 121
<210> 28
   <211> 99
   <212> RNA
   <213> Mus musculus
<400> 28
   cugauucugc cugcguggag cgggcacagc ugugagagcc cccuagguac agcggggcug 60
   cagcgugauc gccugcucac gcacaggaag ugacgacag 99
<210> 29
   <211> 76
   <212> RNA
   <213> Mus musculus
<400> 29
   ugcuuaauga gaaguugccc gcguguuuuu cgcuuuauau gugacgaaac auucgcggug 60
   cacuucuuuu ucagca 76
<210> 30
   <211> 63
   <212> RNA
   <213> Mus musculus
<400> 30
   aaagaaguug cccauguuau uuuucgcuuu uauuugugac gaaacaaaca uggugcacuu 60
   cuu 63
<210> 31
   <211> 92
   <212> RNA
   <213> Mus musculus
<400> 31
   guggguacug gccucggugc ugguggagca gugagcacgc cauacauuau aucugugaca 60
   ccugccaccc agcccaaggc cccuaggccc ac 92
<210> 32
   <211> 86
   <212> RNA
   <213> Mus musculus
<400> 32
   uuugguauuu aaaaggugga uauuccuucu auguuuaugc uuuuugugau uaaacauaga 60
   ggaaauuuca cguuuucagu gucaaa 86
<210> 33
   <211> 84
   <212> RNA
   <213> Mus musculus
<400> 33
   cucgguaagu gggaagaugg uaagcugcag aacaugugug uuucucaugu cauaugucug 60
   cugaccauca ccuuuggguc ucug 84
<210> 34
   <211> 82
   <212> RNA
   <213> Mus musculus
<400> 34
   ugguauuuaa aagguggaua uuccuucuau gguuacgugc uuccuggaua aucauagagg 60
   aacauccacu uuuucaguau ca 82
<210> 35
   <211> 68
   <212> RNA
   <213> Mus musculus
<400> 35
   uaaaagguag auucuccuuc uaugaguaca auauuaauga cuaaucguag aggaaaaucc 60
   acguuuuc 68
<210> 36
   <211> 79
   <212> RNA
   <213> Mus musculus
<400> 36
   gcuacuugaa gagagguuau ccuuugugug uuugcuuuac gcgaaaugaa uaugcaaggg 60
   caagcucucu ucgaggagc 79
<210> 37
   <211> 75
   <212> RNA
   <213> Mus musculus
<400> 37
   uacuuaaagc gagguugccc uuuguauauu cgguuuauug acauggaaua uacaagggca 60
   agcucucugu gagua 75
<210> 38
   <211> 82
   <212> RNA
   <213> Mus musculus
<400> 38
   ugguacuugg agagugguua ucccuguccu cuucgcuuca cucaugccga aucguacagg 60
   gucauccacu uuuucaguau ca 82
<210> 39
   <211> 74
   <212> RNA
   <213> Mus musculus
<400> 39
   uacuugagga gaaauuaucc uugguguguu ggcucuuuug gaugaaucau acaaggauaa    60
   uuucuuuuug agua 74
<210> 40
   <211> 78
   <212> RNA
   <213> Mus musculus
<400> 40
   caccuaggga ucuuguuaaa aagcagaguc ugauugaggg gccaagauuc ugcauuuuua 60
   gcaagcucuc aagugaug 78
<210> 41
   <211> 76
   <212> RNA
   <213> Mus musculus
<400> 41
   uacuugaaga gaaguuguuc gugguggauu cgcuuuacuu gugacgaauc auucacggac 60
   aacacuuuuu ucagua 76
<210> 42
   <211> 71
   <212> RNA
   <213> Mus musculus
<400> 42
   agggugugug acugguugac cagaggggcg ugcacucugu ucacccugug ggccaccuag 60
   ucaccaaccc u 71
<210> 43
   <211> 66
   <212> RNA
   <213> Mus musculus
<400> 43
   gguaagugug ccucggguga gcaugcacuu aauguaggug uaugucacuc ggcucggccc 60
   acuacc 66
<210> 44
   <211> 73
   <212> RNA
   <213> Mus musculus
<400> 44
   acuuggagag aggcuggccg ugaugaauuc gauucaucua aacgagucau acacggcucu 60
   ccucucuucu agu 73
<210> 45
   <211> 82
   <212> RNA
   <213> Mus musculus
<400> 45
   agaagaugca ggagugcugu gagaagugcc auccccuggu acuuggaggg agguugccuc 60
   auagugagcu ugcauuauuu aa 82
<210> 46
   <211> 66
   <212> RNA
   <213> Mus musculus
<400> 46
   gaagauaggu uauccguguu gccuucgcuu uauucgugac gaaucauaca cgguugaccu 60
   auuuuu 66
<210> 47
   <211> 79
   <212> RNA
   <213> Mus musculus
<400> 47
   aguguucgaa uggagguugc ccauggugug uucauuuuau uuaugaugag uauuacaugg 60
   ccaaucuccu uucggcacu 79
<210> 48
   <211> 68
   <212> RNA
   <213> Mus musculus
<400> 48
   ugagcagagg uugcccuugg ugaauucgcu uuauugaugu ugaaucacac aaaggcaacu 60
   uuuguuug 68
<210> 49
   <211> 90
   <212> RNA
   <213> Mus musculus
<400> 49
   gccaaaauca gagaagggau ucugauguug gucacacucc aagaguuuua aaaugagugg 60
   cgaacacaga auccauacuc ugcuuauggc 90
<210> 50
   <211> 79
   <212> RNA
   <213> Mus musculus
<400> 50
   ugguacucgg agagagguua cccgagcaac uuugcaucug gaggacgaau guugcucggu 60
   gaaccccuuu ucgguauca 79
<210> 51
   <211> 80
   <212> RNA
   <213> Mus musculus
<400> 51
   ggguauggga cggauggucg accagcugga aaguaauugu uucuaaugua cuucaccugg 60
   uccacuagcc gucggugccc 80
<210> 52
   <211> 79
   <212> RNA
   <213> Mus musculus
<400> 52
   gguacuugaa gggagaucga ccguguuaua uucgcuuggc ugacuucgaa uaauacaugg 60
   uugaucuuuu cucaguauc 79
<210> 53
   <211> 81
   <212> RNA
   <213> Mus musculus
<400> 53
   ggguacuuga ggagagguug ucugugauga guucgcuuua uuaaugacga auauaacaca 60
   gauggccugu uuucaauacc a 81
<210> 54
   <211> 22
   <212> RNA
   <213> Mus musculus
<400> 54
   cgugggccug acguggagcu gg 22
<210> 55
   <211> 22
   <212> RNA
   <213> Mus musculus
<400> 55
   agcaccacgu gucugggcca cg 22
<210> 56
   <211> 23
   <212> RNA
   <213> Mus musculus
<400> 56
   uccggggcug aguucugugc acc 23
<210> 57
   <211> 22
   <212> RNA
   <213> Mus musculus
<400> 57
   cucacagcuc ugguccuugg ag 22
<210> 58
   <211> 23
   <212> RNA
   <213> Mus musculus
<400> 58
   ugaagguccu acugugugcc agg 23
<210> 59
   <211> 21
   <212> RNA
   <213> Mus musculus
<400> 59
   uucagcuccu auaugaugcc u 21
<210> 60
   <211> 21
   <212> RNA
   <213> Mus musculus
<400> 60
   gaacggcguc augcaggagu u 21
<210> 61
   <211> 20
   <212> RNA
   <213> Mus musculus
<400> 61
   aggucagagg ucgauccugg 20
<210> 62
   <211> 23
   <212> RNA
   <213> Mus musculus
<400> 62
   caagggucac ccucugacuc ugu 23
<210> 63
   <211> 20
   <212> RNA
   <213> Mus musculus
<400> 63
   accaggaggc ugaggucccu 20
<210> 64
   <211> 21
   <212> RNA
   <213> Mus musculus
<400> 64
   ugucuugcag gccgucaugc a 21
<210> 65
   <211> 22
   <212> RNA
   <213> Mus musculus
<400> 65
   caggucgucu ugcagggcuu cu 22
<210> 66
   <211> 22
   <212> RNA
   <213> Mus musculus
<400> 66
   aucaugaugg gcuccucggu gu 22
<210> 67
   <211> 22
   <212> RNA
   <213> Mus musculus
<400> 67
   uacggugagc cugucauuau uc 22
<210> 68
   <211> 22
   <212> RNA
   <213> Mus musculus
<400> 68
   ucggauccgu cugagcuugg cu 22
<210> 69
   <211> 22
   <212> RNA
   <213> Mus musculus
<400> 69
   cugaagcuca gagggcucug au 22
<210> 70
   <211> 22
   <212> RNA
   <213> Mus musculus
<400> 70
   uuugaaccau cacucgacuc cu 22
<210> 71
   <211> 22
   <212> RNA
   <213> Mus musculus
<400> 71
   gcucgacuca ugguuugaac ca 22
<210> 72
   <211> 23
   <212> RNA
   <213> Mus musculus
<400> 72
   ucuuggagua gaucaguggg cag 23
<210> 73
   <211> 22
   <212> RNA
   <213> Mus musculus
<400> 73
   acuccauuug uuuugaugau gg 22
<210> 74
   <211> 22
   <212> RNA
   <213> Mus musculus
<400> 74
   aucaucgucu caaaugaguc uu 22
<210> 75
   <211> 21
   <212> RNA
   <213> Mus musculus
<400> 75
   ucggucgauc ggucggucgg u 21
<210> 76
   <211> 21
   <212> RNA
   <213> Mus musculus
<400> 76
   uggugugagg uugggccagg a 21
<210> 77
   <211> 22
   <212> RNA
   <213> Mus musculus
<400> 77
   gccugcuggg guggaaccug gu 22
<210> 78
   <211> 22
   <212> RNA
   <213> Mus musculus
<400> 78
   aggagagagu uagcgcauua gu 22
<210> 79
   <211> 21
   <212> RNA
   <213> Mus musculus
<400> 79
   ugguagacua uggaacguag g 21
<210> 80
   <211> 21
   <212> RNA
   <213> Mus musculus
<400> 80
   uaguagaccg uauagcguac g 21
<210> 81
   <211> 22
   <212> RNA
   <213> Mus musculus
<400> 81
   uauguaacac gguccacuaa cc 22
<210> 82
   <211> 22
   <212> RNA
   <213> Mus musculus
<400> 82
   uaugugggac gguaaaccgc uu 22
<210> 83
   <211> 22
   <212> RNA
   <213> Mus musculus
<400> 83
   ugguuuaccg ucccacauac au 22
<210> 84
   <211> 22
   <212> RNA
   <213> Mus musculus
<400> 84
   uauguaguau gguccacauc uu 22
<210> 85
   <211> 22
   <212> RNA
   <213> Mus musculus
<400> 85
   augguugacc auagaacaug cg 22
<210> 86
   <211> 21
   <212> RNA
   <213> Mus musculus
<400> 86
   uaggacacau ggucuacuuc u 21
<210> 87
   <211> 21
   <212> RNA
   <213> Mus musculus
<400> 87
   cacauuacac ggucgaccuc u 21
<210> 88
   <211> 22
   <212> RNA
   <213> Mus musculus
<400> 88
   aggugguccg uggcgcguuc gc 22
<210> 89
   <211> 19
   <212> RNA
   <213> Mus musculus
<400> 89
   uuugugaccu gguccacua 19
<210> 90
   <211> 22
   <212> RNA
   <213> Mus musculus
<400> 90
   aacacaccca gcuaaccuuu uu 22
<210> 91
   <211> 22
   <212> RNA
   <213> Mus musculus
<400> 91
   ugaaacauac acgggaaacc uc 22
<210> 92
   <211> 22
   <212> RNA
   <213> Mus musculus
<400> 92
   ggacugugag gugacucuug gu 22
<210> 93
   <211> 21
   <212> RNA
   <213> Mus musculus
<400> 93
   uaggucaccc guuuuacuau c 21
<210> 94
   <211> 22
   <212> RNA
   <213> Mus musculus
<400> 94
   ggcugcagcg ugaucgccug cu 22
<210> 95
   <211> 22
   <212> RNA
   <213> Mus musculus
<400> 95
   agcgggcaca gcugugagag cc 22
<210> 96
   <211> 22
   <212> RNA
   <213> Mus musculus
<400> 96
   aaacauucgc ggugcacuuc uu 22
<210> 97
   <211> 22
   <212> RNA
   <213> Mus musculus
<400> 97
   aaacaaacau ggugcacuuc uu 22
<210> 98
   <211> 23
   <212> RNA
   <213> Mus musculus
<400> 98
   ugacaccugc cacccagccc aag 23
<210> 99
   <211> 21
   <212> RNA
   <213> Mus musculus
<400> 99
   aacauagagg aaauuucacg u 21
<210> 100
   <211> 22
   <212> RNA
   <213> Mus musculus
<400> 100
   guggauauuc cuucuauguu ua 22
<210> 101
   <211> 22
   <212> RNA
   <213> Mus musculus
<400> 101
   uaugucugcu gaccaucacc uu 22
<210> 102
   <211> 22
   <212> RNA
   <213> Mus musculus
<400> 102
   ugguaagcug cagaacaugu gu 22
<210> 103
   <211> 21
   <212> RNA
   <213> Mus musculus
<400> 103
   aucauagagg aacauccacu u 21
<210> 104
   <211> 22
   <212> RNA
   <213> Mus musculus
<400> 104
   guggauauuc cuucuauggu ua 22
<210> 105
   <211> 21
   <212> RNA
   <213> Mus musculus
<400> 105
   aucguagagg aaaauccacg u 21
<210> 106
   <211> 22
   <212> RNA
   <213> Mus musculus
<400> 106
   gguagauucu ccuucuauga gu 22
<210> 107
   <211> 22
   <212> RNA
   <213> Mus musculus
<400> 107
   uaugcaaggg caagcucucu uc 22
<210> 108
   <211> 22
   <212> RNA
   <213> Mus musculus
<400> 108
   uugaagagag guuauccuuu gu 22
<210> 109
   <211> 22
   <212> RNA
   <213> Mus musculus
<400> 109
   uauacaaggg caagcucucu gu 22
<210> 110
   <211> 22
   <212> RNA
   <213> Mus musculus
<400> 110
   aaucguacag ggucauccac uu 22
<210> 111
   <211> 22
   <212> RNA
   <213> Mus musculus
<400> 111
   ggagaaauua uccuuggugu gu 22
<210> 112
   <211> 22
   <212> RNA
   <213> Mus musculus
<400> 112
   auucugcauu uuuagcaagc uc 22
<210> 113
   <211> 22
   <212> RNA
   <213> Mus musculus
<400> 113
   gaaguuguuc gugguggauu cg 22
<210> 114
   <211> 22
   <212> RNA
   <213> Mus musculus
<400> 114
   ucauucacgg acaacacuuu uu 22
<210> 115
   <211> 22
   <212> RNA
   <213> Mus musculus
<400> 115
   ugugacuggu ugaccagagg gg 22
<210> 116
   <211> 24
   <212> RNA
   <213> Mus musculus
<400> 116
   ugucacucgg cucggcccac uacc 24
<210> 117
   <211> 22
   <212> RNA
   <213> Mus musculus
<400> 117
   agaggcuggc cgugaugaau uc 22
<210> 118
   <211> 22
   <212> RNA
   <213> Mus musculus
<400> 118
   agucauacac ggcucuccuc uc 22
<210> 119
   <211> 24
   <212> RNA
   <213> Mus musculus
<400> 119
   agguugccuc auagugagcu ugca 24
<210> 120
   <211> 22
   <212> RNA
   <213> Mus musculus
<400> 120
   uagguuaucc guguugccuu cg 22
<210> 121
   <211> 22
   <212> RNA
   <213> Mus musculus
<400> 121
   aaucauacac gguugaccua uu 22
<210> 122
   <211> 22
   <212> RNA
   <213> Mus musculus
<400> 122
   ugaguauuac.auggccaauc uc 22
<210> 123
   <211> 22
   <212> RNA
   <213> Mus musculus
<400> 123
   aucacacaaa ggcaacuuuu gu 22
<210> 124
   <211> 25
   <212> RNA
   <213> Mus musculus
<400> 124
   aagggauucu gauguugguc acacu 25
<210> 125
   <211> 22
   <212> RNA
   <213> Mus musculus
<400> 125
   gaauguugcu cggugaaccc cu 22
<210> 126
   <211> 23
   <212> RNA
   <213> Mus musculus
<400> 126
   agguuacccg agcaacuuug cau 23
<210> 127
   <211> 20
   <212> RNA
   <213> Mus musculus
<400> 127
   uucaccuggu ccacuagccg 20
<210> 128
   <211> 21
   <212> RNA
   <213> Mus musculus
<400> 128
   aauaauacau gguugaucuu u 21
<210> 129
   <211> 22
   <212> RNA
   <213> Mus musculus
<400> 129
   agaucgaccg uguuauauuc gc 22
<210> 130
   <211> 21
   <212> RNA
   <213> Mus musculus
<400> 130
   aauauaacac agauggccug u 21
<210> 131
   <211> 98
   <212> RNA
   <213> Homo sapiens
<400> 131
   aggagccacc uuccgagccu ccaguaccac gugucagggc cacaugagcu gggccucgug 60
   ggccugaugu ggugcugggg ccucaggggu cugcucuu 98
<210> 132
   <211> 89
   <212> RNA
   <213> Homo sapiens
<400> 132
   cuggccucca gggcuuugua caugguaggc uuucauucau ucguuugcac auucggugaa 60
   ggucuacugu gugccaggcc cugugccag 89
<210> 133
   <211> 93
   <212> RNA
   <213> Homo sapiens
<400> 133
   guagucagua guuggggggu gggaacggcu ucauacagga guugaugcac aguuauccag 60
   cuccuauaug augccuuucu ucauccccuu caa 93
<210> 134
   <211> 72
   <212> RNA
   <213> Homo sapiens
<400> 134
   ucuccucgag gggucucugc cucuacccag gacucuuuca ugaccaggag gcugaggccc 60
   cucacaggcg gc 72
<210> 135
   <211> 114
   <212> RNA
   <213> Homo sapiens
<400> 135
   uccugcuugu ccugcgaggu gucuugcagg ccgucaugca ggccacacug acgguaacgu 60
   ugcaggucgu cuugcagggc uucucgcaag acgacauccu caucaccaac gacg 114
<210> 136
   <211> 93
   <212> RNA
   <213> Homo sapiens
<400> 136
   ccggggagaa guacggugag ccugucauua uucagagagg cuagauccuc uguguugaga 60
   aggaucauga ugggcuccuc gguguucucc agg 93
<210> 137
   <211> 97
   <212> RNA
   <213> Homo sapiens
<400> 137
   ugugaucacu gucuccagcc ugcugaagcu cagagggcuc ugauucagaa agaucaucgg 60
   auccgucuga gcuuggcugg ucggaagucu caucauc 97
<210> 138
   <211> 94
   <212> RNA
   <213> Homo sapiens
<400> 138
   ugacuccucc aggucuugga guaggucauu ggguggaucc ucuauuuccu uacgugggcc 60
   acuggauggc uccuccaugu cuuggaguag auca 94
<210> 139
   <211> 82
   <212> RNA
   <213> Homo sapiens
<400> 139
   ugagcccucg gaggacucca uuuguuuuga ugauggauuc uuaugcucca ucaucgucuc 60
   aaaugagucu ucagaggguu cu 82
<210> 140
   <211> 75
   <212> RNA
   <213> Homo sapiens
<400> 140
   agacagagaa gccaggucac gucucugcag uuacacagcu cacgagugcc ugcuggggug 60
   gaaccugguc ugucu 75
<210> 141
   <211> 67
   <212> RNA
   <213> Homo sapiens
<400> 141
   agagauggua gacuauggaa cguaggcguu augauuucug accuauguaa caugguccac 60
   uaacucu 67
<210> 142
   <211> 96
   <212> RNA
   <213> Homo sapiens
<400> 142
   ugguacuugg agagauagua gaccguauag cguacgcuuu aucugugacg uauguaacac 60
   gguccacuaa cccucaguau caaauccauc cccgag 96
<210> 143
   <211> 63
   <212> RNA
   <213> Homo sapiens
<400> 143
   aagaaauggu uuaccguccc acauacauuu ugaauaugua ugugggaugg uaaaccgcuu 60
   cuu 63
<210> 144
   <211> 61
   <212> RNA
   <213> Homo sapiens
<400> 144
   aagaugguug accauagaac augcgcuauc ucugugucgu auguaauaug guccacaucu 60
   u 61
<210> 145
   <211> 88
   <212> RNA
   <213> Homo sapiens
<400> 145
   acuuccuggu auuugaagau gcgguugacc auggugugua cgcuuuauuu gugacguagg 60
   acacaugguc uacuucuucu caauauca 88
<210> 146
   <211> 86
   <212> RNA
   <213> Homo sapiens
<400> 146
   uugguacuug gagagaggug guccguggcg cguucgcuuu auuuauggcg cacauuacac 60
   ggucgaccuc uuugcaguau cuaauc 86
<210> 147
   <211> 88
   <212> RNA
   <213> Homo sapiens
<400> 147
   gccuggauac augagauggu ugaccagaga gcacacgcuu uauuugugcc guuugugacc 60
   ugguccacua acccucagua ucuaaugc 88
<210> 148
   <211> 80
   <212> RNA
   <213> Homo sapiens
<400> 148
   gguaccugaa gagagguuuu cuggguuucu guuucuuuaa ugaggacgaa acacaccugg 60
   uuaaccucuu uuccaguauc 80
<210> 149
   <211> 84
   <212> RNA
   <213> Homo sapiens
<400> 149
   gugguaccug aagagagguu uucuggguuu cuguuucuuu auugaggacg aaacacaccu 60
   gguuaaccuc uuuuccagua ucaa 84
<210> 150
   <211> 81
   <212> RNA
   <213> Homo sapiens
<400> 150
   gauacucgaa ggagagguug uccguguugu cuucucuuua uuuaugauga aacauacacg 60
   ggaaaccucu uuuuuaguau c 81
<210> 151
   <211> 78
   <212> RNA
   <213> Homo sapiens
<400> 151
   uacuuaauga gaaguugccc guguuuuuuu cgcuuuauuu gugacgaaac auucgcggug 60
   cacuucuuuu ucaguauc 78
<210> 152
   <211> 82
   <212> RNA
   <213> Homo sapiens
<400> 152
   ugguaccuga aaagaaguug cccauguuau uuucgcuuua uaugugacga aacaaacaug 60
   gugcacuucu uuuucgguau ca 82
<210> 153
   <211> 66
   <212> RNA
   <213> Homo sapiens
<400> 153
   aaaaggugga uauuccuucu auguuuaugu uauuuauggu uaaacauaga ggaaauucca 60
   cguuuu 66
<210> 154
   <211> 80
   <212> RNA
   <213> Homo sapiens
<400> 154
   gguauuuaaa agguagauuu uccuucuaug guuacguguu ugaugguuaa ucauagagga 60
   aaauccacgu uuucaguauc 80
<210> 155
   <211> 81
   <212> RNA
   <213> Homo sapiens
<400> 155
   ggguaagugg aaagauggug ggccgcagaa caugugcuga guucgugcca uaugucugcu 60
   gaccaucacc uuuagaagcc c 81
<210> 156
   <211> 100
   <212> RNA
   <213> Homo sapiens
<400> 156
   caguccuucu uugguauuua aaacguggau auuccuucua uguuuacgug auuccugguu 60
   aaucauagag gaaaauccau guuuucagua ucaaaugcug 100
<210> 157
   <211> 68
   <212> RNA
   <213> Homo sapiens
<400> 157
   uaaaagguag auucuccuuc uaugaguaca uuauuuauga uuaaucauag aggaaaaucc 60
   acguuuuc 68
<210> 158
   <211> 83
   <212> RNA
   <213> Homo sapiens
<400> 158
   ugcuacuuga agagagguaa uccuucacgc auuugcuuua cuugcaauga uuauacaagg 60
   gcagacucuc ucuggggagc aaa 83
<210> 159
   <211> 86
   <212> RNA
   <213> Homo sapiens
<400> 159
   uuugguacuu gaagagagga uacccuuugu auguucacuu gauuaauggc gaauauacag 60
   ggggagacuc uuauuugcgu aucaaa 86
<210> 160
   <211> 86
   <212> RNA
   <213> Homo sapiens
<400> 160
   uuugguacuu aaagagagga uacccuuugu auguucacuu gauuaauggc gaauauacag 60
   ggggagacuc ucauuugcgu aucaaa 86
<210> 161
   <211> 75
   <212> RNA
   <213> Homo sapiens
<400> 161
   uacuuaaagc gagguugccc uuuguauauu cgguuuauug acauggaaua uacaagggca 60
   agcucucugu gagua 75
<210> 162
   <211> 84
   <212> RNA
   <213> Homo sapiens
<400> 162
   uugguacuug gagagugguu aucccugucc uguucguuuu gcucaugucg aaucguacag 60
   ggucauccac uuuuucagua ucaa 84
<210> 163
   <211> . 78
   <212> RNA
   <213> Homo sapiens
<400> 163
<210> 164
   <211> 79
   <212> RNA
   <213> Homo sapiens
<400> 164
   gugcuuaaag aauggcuguc cguaguaugg ucucuauauu uaugaugauu aauaucggac 60
   aaccauuguu uuaguaucc 79
<210> 165
   <211> 91
   <212> RNA
   <213> Homo sapiens
<400> 165
   auuuucauca ccuagggauc uuguuaaaaa gcagauucug auucagggac caagauucug 60
   cauuuuuagc aaguucucaa gugaugcuaa u 91
<210> 166
   <211> 97
   <212> RNA
   <213> Homo sapiens
<400> 166
   aacuaugcaa ggauauuuga ggagagguua uccguguuau guucgcuuca uucaucauga 60
   auaauacaug guuaaccucu uuuugaauau cagacuc 97
<210> 167
   <211> 80
   <212> RNA
   <213> Homo sapiens
<400> 167
   gguacuugaa gagugguuau cccugcugug uucgcuuaau uuaugacgaa ucauacaggg 60
   acauccaguu uuucaguauc 80
<210> 168
   <211> 76
   <212> RNA
   <213> Homo sapiens
<400> 168
   uacuugaaga gaaguuguuc gugguggauu cgcuuuacuu augacgaauc auucacggac 60
   aacacuuuuu ucagua 76
<210> 169
   <211> 73
   <212> RNA
   <213> Homo sapiens
<400> 169
   cagggugugu gacugguuga ccagaggggc augcacugug uucacccugu gggccaccua 60
   gucaccaacc cuc 73
<210> 170
   <211> 66
   <212> RNA
   <213> Homo sapiens
<400> 170
   gguaagugcg ccucggguga gcaugcacuu aaugugggug uaugucacuc ggcucggccc 60
   acuacc 66
<210>. 171
   <211> 73
   <212> RNA
   <213> Homo sapiens
<400> 171
   acuuggagag aggcuggccg ugaugaauuc gauucaucaa agcgagucau acacggcucu 60
   ccucucuuuu agu 73
<210> 172
   <211> 80
   <212> RNA
   <213> Homo sapiens
<400> 172
   gcaggaaugc ugcgagcagu gccaccucau gguacucgga gggagguugu ccguggugag 60
   uucgcauuau uuaaugaugc 80
<210> 173
   <211> 84
   <212> RNA
   <213> Homo sapiens
<400> 173
   gugguacuug aagauagguu auccguguug ccuucgcuuu auuugugacg aaucauacac 60
   gguugaccua uuuuucagua ccaa 84
<210> 174
   <211> 102
   <212> RNA
   <213> Homo sapiens
<400> 174
   cccaagucag guacucgaau ggagguuguc cauggugugu ucauuuuauu uaugaugagu 60
   auuacauggc caaucuccuu ucgguacuca auucuucuug gg 102
<210> 175
   <211> 69
   <212> RNA
   <213> Homo sapiens
<400> 175
   uugagcagag guugcccuug gugaauucgc uuuauuuaug uugaaucaca caaaggcaac 60
   uuuuguuug 69
<210> 176
   <211> 84
   <212> RNA
   <213> Homo sapiens
<400> 176
   acgucaggga aaggauucug cugucggucc cacuccaaag uucacagaau gggugguggg    60
   cacagaaucu ggacucugcu ugug 84
<210> 177
   <211> 79
   <212> RNA
   <213> Homo sapiens
<400> 177
   ugguacucgg ggagagguua cccgagcaac uuugcaucug gacgacgaau guugcucggu 60
   gaaccccuuu ucgguauca 79
<210> 178
   <211> 91
   <212> RNA
   <213> Homo sapiens
<400> 178
   cugggguacg gggauggaug gucgaccagu uggaaaguaa uuguuucuaa uguacuucac 60
   cugguccacu agccguccgu auccgcugca g 91
<210> 179
   <211> 70
   <212> RNA
   <213> Homo sapiens
<400> 179
   uugaagggag aucgaccgug uuauauucgc uuuauugacu ucgaauaaua caugguugau 60
   cuuuucucag 70
<210> 180
   <211> 80
   <212> RNA
   <213> Homo sapiens
<400> 180
   gguaccugag aagagguugu cugugaugag uucgcuuuua uuaaugacga auauaacaca 60
   gauggccugu uuucaguacc 80
<210> 181
   <211> 78
   <212> RNA
   <213> Homo sapiens
<400> 181
   cugaaauagg uugccuguga gguguucacu uucuauauga ugaauauuau acagucaacc 60
   ucuuuccgau aucgaauc 78
<210> 182
   <211> 23
   <212> RNA
   <213> Homo sapiens
<400> 182
   uccaguacca cgugucaggg cca 23
<210> 183
   <211> 22
   <212> RNA
   <213> Homo sapiens
<400> 183
   ugaaggucua cugugugcca gg 22
<210> 184
   <211> 22
   <212> RNA
   <213> Homo sapiens
<400> 184
   uuguacaugg uaggcuuuca uu 22
<210> 185
   <211> 21
   <212> RNA
   <213> Homo sapiens
<400> 185
   gaacggcuuc auacaggagu u 21
<210> 186
   <211> 22
   <212> RNA
   <213> Homo sapiens
<400> 186
   cuccuauaug augccuuucu uc 22
<210> 187
   <211> 20
   <212> RNA
   <213> Homo sapiens
<400> 187
   accaggaggc ugaggccccu 20
<210> 188
   <211> 21
   <212> RNA
   <213> Homo sapiens
<400> 188
   ugucuugcag gccgucaugc a 21
<210> 189
   <211> 22
   <212> RNA
   <213> Homo sapiens
<400> 189
   caggucgucu ugcagggcuu cu 22
<210> 190
   <211> 22
   <212> RNA
   <213> Homo sapiens
<400> 190
   aucaugaugg gcuccucggu gu 22
<210> 191
   <211> 22
   <212> RNA
   <213> Homo sapiens
<400> 191
   cugaagcuca gagggcucug au 22
<210> 192
   <211> 22
   <212> RNA
   <213> Homo sapiens
<400> 192
   ucggauccgu cugagcuugg cu 22
<210> 193
   <211> 23
   <212> RNA
   <213> Homo sapiens
<400> 193
   ucuuggagua ggucauuggg ugg 23
<210> 194
   <211> 21
   <212> RNA
   <213> Homo sapiens
<400> 194
   cuggauggcu ccuccauguc u 21
<210> 195
   <211> 23
   <212> RNA
   <213> Homo sapiens
<400> 195
   acuccauuug uuuugaugau gga 23
<210> 196
   <211> 22
   <212> RNA
   <213> Homo sapiens
<400> 196
   caucaucguc ucaaaugagu cu 22
<210> 197
   <211> 22
   <212> RNA
   <213> Homo sapiens
<400> 197
   gccugcuggg guggaaccug gu 22
<210> 198
   <211> 21
   <212> RNA
   <213> Homo sapiens
<400> 198
   ugguagacua uggaacguag g 21
<210> 199
   <211> 22
   <212> RNA
   <213> Homo sapiens
<400> 199
   uauguaacau gguccacuaa cu 22
<210> 200
   <211> 21
   <212> RNA
   <213> Homo sapiens
<400> 200
   uaguagaccg uauagcguac g 21
<210> 201
   <211> 22
   <212> RNA
   <213> Homo sapiens
<400> 201
   uauguaacac gguccacuaa cc 22
<210> 202
   <211> 22
   <212> RNA
   <213> Homo sapiens
<400> 202
   ugguuuaccg ucccacauac au 22
<210> 203
   <211> 22
   <212> RNA
   <213> Homo sapiens
<400> 203
   uaugugggau gguaaaccgc uu 22
<210> 204
   <211> 22
   <212> RNA
   <213> Homo sapiens
<400> 204
   uauguaauau gguccacauc uu 22
<210> 205
   <211> 22
   <212> RNA
   <213> Homo sapiens
<400> 205
   ugguugacca uagaacaugc gc 22
<210> 206
   <211> 21
   <212> RNA
   <213> Homo sapiens
<400> 206
   uaggacacau ggucuacuuc u 21
<210> 207
   <211> 22
   <212> RNA
   <213> Homo sapiens
<400> 207
   aggugguccg uggcgcguuc gc 22
<210> 208
   <211> 21
   <212> RNA
   <213> Homo sapiens
<400> 208
   cacauuacac ggucgaccuc u 21
<210> 209
   <211> 22
   <212> RNA
   <213> Homo sapiens
<400> 209
   uuugugaccu gguccacuaa cc 22
<210> 210
   <211> 22
   <212> RNA
   <213> Homo sapiens
<400> 210
   aacacaccug guuaaccucu uu 22
<210> 211
   <211> 22
   <212> RNA
   <213> Homo sapiens
<400> 211
   ugaaacauac acgggaaacc uc 22
<210> 212
   <211> 22
   <212> RNA
   <213> Homo sapiens
<400> 212
   aaacauucgc ggugcacuuc uu 22
<210> 213
   <211> 22
   <212> RNA
   <213> Homo sapiens
<400> 213
   aaacaaacau ggugcacuuc uu 22
<210> 214
   <211> 21
   <212> RNA
   <213> Homo sapiens
<400> 214
   aacauagagg aaauuccacg u 21
<210> 215
   <211> 21
   <212> RNA
   <213> Homo sapiens
<400> 215
   aucauagagg aaaauccacg u 21
<210> 216
   <211> 22
   <212> RNA
   <213> Homo sapiens
<400> 216
   uggugggccg cagaacaugu gc 22
<210> 217
   <211> 22
   <212> RNA
   <213> Homo sapiens
<400> 217
   uaugucugcu gaccaucacc uu 22
<210> 218
   <211> 22
   <212> RNA
   <213> Homo sapiens
<400> 218
   aucauagagg aaaauccaug uu 22
<210> 219
   <211> 21
   <212> RNA
   <213> Homo sapiens
<400> 219
   aucauagagg aaaauccacg u 21
<210> 220
   <211> 22
   <212> RNA
   <213> Homo sapiens
<400> 220
   guagauucuc cuucuaugag ua 22
<210> 221
   <211> 22
   <212> RNA
   <213> Homo sapiens
<400> 221
   uauacaaggg cagacucucu cu 22
<210> 222
   <211> 21
   <212> RNA
   <213> Homo sapiens
<400> 222
   agaggauacc cuuuguaugu u 21
<210> 223
   <211> 22
   <212> RNA
   <213> Homo sapiens
<400> 223
   uauacaaggg caagcucucu gu 22
<210> 224
   <211> 22
   <212> RNA
   <213> Homo sapiens
<400> 224
   aaucguacag ggucauccac uu 22
<210> 225
   <211> 22
   <212> RNA
   <213> Homo sapiens
<400> 225
   ggagaaauua uccuuggugu gu 22
<210> 226
   <211> 21
   <212> RNA
   <213> Homo sapiens
<400> 226
   uuaauaucgg acaaccauug u 21
<210> 227
   <211> 22
   <212> RNA
   <213> Homo sapiens
<400> 227
   auucugcauu uuuagcaagu uc 22
<210> 228
   <211> 22
   <212> RNA
   <213> Homo sapiens
<400> 228
   auaauacaug guuaaccucu uu 22
<210> 229
   <211> 22
   <212> RNA
   <213> Homo sapiens
<400> 229
   aaucauacag ggacauccag uu 22
<210> 230
   <211> 22
   <212> RNA
   <213> Homo sapiens
<400> 230
   gaaguuguuc gugguggauu cg 22
<210> 231
   <211> 22
   <212> RNA
   <213> Homo sapiens
<400> 231
   ugugacuggu ugaccagagg gg 22
<210> 232
   <211> 23
   <212> RNA
   <213> Homo sapiens
<400> 232
   ugucacucgg cucggcccac uac 23
<210> 233
   <211> 22
   <212> RNA
   <213> Homo sapiens
<400> 233
   agaggcuggc cgugaugaau uc 22
<210> 234
   <211> 22
   <212> RNA
   <213> Homo sapiens
<400> 234
   gucauacacg gcucuccucu cu 22
<210> 235
   <211> 23
   <212> RNA
   <213> Homo sapiens
<400> 235
   agguuguccg uggugaguuc gca 23
<210> 236
   <211> 22
   <212> RNA
   <213> Homo sapiens
<400> 236
   uagguuaucc guguugccuu cg 22
<210> 237
   <211> 22
   <212> RNA
   <213> Homo sapiens
<400> 237
   aaucauacac gguugaccua uu 22
<210> 238
   <211> 22
   <212> RNA
   <213> Homo sapiens
<400> 238
   ugaguauuac auggccaauc uc 22
<210> 239
   <211> 22
   <212> RNA
   <213> Homo sapiens
<400> 239
   aucacacaaa ggcaacuuuu gu 22
<210> 240
   <211> 22
   <212> RNA
   <213> Homo sapiens
<400> 240
   agagguugcc cuuggugaau uc 22
<210> 241
   <211> 22
   <212> RNA
   <213> Homo sapiens
<400> 241
   uggugggcac agaaucugga cu 22
<210> 242
   <211> 25
   <212> RNA
   <213> Homo sapiens
<400> 242
   aaaggauucu gcugucgguc ccacu 25
<210> 243
   <211> 23
   <212> RNA
   <213> Homo sapiens
<400> 243
   agguuacccg agcaacuuug cau 23
<210> 244
   <211> 22
   <212> RNA
   <213> Homo sapiens
<400> 244
   gaauguugcu cggugaaccc cu 22
<210> 245
   <211> 23
   <212> RNA
   <213> Homo sapiens
<400> 245
   acuucaccug guccacuagc cgu 23
<210> 246
   <211> 22
   <212> RNA
   <213> Homo sapiens
<400> 246
   agaucgaccg uguuauauuc gc 22
<210> 247
   <211> 21
   <212> RNA
   <213> Homo sapiens
<400> 247
   aauaauacau gguugaucuu u 21
<210> 248
   <211> 21
   <212> RNA
   <213> Homo sapiens
<400> 248
   aauauaacac agauggccug u 21
<210> 249
   <211> 21
   <212> RNA
   <213> Homo sapiens
<400> 249
   aauauuauac agucaaccuc u 21
<210> 250
   <211> 22
   <212> RNA
   <213> Mus musculus
<400> 250
   acucaaacua ugggggcacu uu 22
<210> 251
   <211> 24
   <212> RNA
   <213> Mus musculus
<400> 251
   aaagugccgc cuaguuuuaa gccc 24
<210> 252
   <211> 22
   <212> RNA
   <213> Mus musculus
<400> 252
   caucaaagug gaggcccucu cu 22
<210> 253
   <211> 22
   <212> RNA
   <213> Mus musculus
<400> 253
   aaagugcuuc cacuuugugu gc 22
<210> 254
   <211> 22
   <212> RNA
   <213> Mus musculus
<400> 254
   acucaaacug ggggcucuuu ug 22
<210> 255
   <211> 24
   <212> RNA
   <213> Mus musculus
<400> 255
   aaagugccgc cagguuuuga gugu 24
<210> 256
   <211> 22
   <212> RNA
   <213> Mus musculus
<400> 256
   agugccgcag aguuuguagu gu 22
<210> 257
   <211> 22
   <212> RNA
   <213> Mus musculus
<400> 257
   acucaaacug ugugacauuu ug 22
<210> 258
   <211> 22
   <212> RNA
   <213> Mus musculus
<400> 258
   aaagugcuuc ccuuuugugu gu 22
<210> 259
   <211> 22
   <212> RNA
   <213> Mus musculus
<400> 259
   acucaaaaug gaggcccuau cu 22
<210> 260
   <211> 23
   <212> RNA
   <213> Mus musculus
<400> 260
   aaagugcuac uacuuuugag ucu 23
<210> 261
   <211> 22
   <212> RNA
   <213> Mus musculus
<400> 261
   acucaaaugu ggggcacacu uc 22
<210> 262
   <211> 23
   <212> RNA
   <213> Homo sapiens
<400> 262
   acuuaaacgu ggauguacuu gcu 23
<210> 263
   <211> 22
   <212> RNA
   <213> Homo sapiens
<400> 263
   aauugcacuu uagcaauggu ga 22
<210> 264
   <211> 23
   <212> RNA
   <213> Homo sapiens
<400> 264
   uaagugcuuc cauguuucag ugg 23
<210> 265
   <211> 23
   <212> RNA
   <213> Homo sapiens
<400> 265
   uaagugcuuc cauguuugag ugu 23
<210> 266
   <211> 22
   <212> RNA
   <213> Homo sapiens
<400> 266
   uuuaacaugg ggguaccugc ug 22
<210> 267
   <211> 22
   <212> RNA
   <213> Homo sapiens
<400> 267
   acuuuaacau ggaagugcuu uc 22
<210> 268
   <211> 23
   <212> RNA
   <213> Homo sapiens
<400> 268
   uaagugcuuc cauguuuugg uga 23
<210> 269
   <211> 23
   <212> RNA
   <213> Homo sapiens
<400> 269
   uaagugcuuc cauguuuuag uag 23
<210> 270
   <211> 11439
   <212> DNA
   <213> Mus musculus
<400> 270
   ggaaagccag gttgtctacc ccacagagcg cttctgaaga ccaaactaca taactcacaa 60
   gaaagtgcct tgtaaatcgc ccggaaattg caaaaaaaaa atccttaatt agccaatgag 120
   gctgtcccta ctgcccagcg gccagcccta gtctctcaca acagtgagca cccagtaggt 180
   gctgggtgtc tttgtgttca acaaataatt tccagaattc aaacaaacaa acaaacaaac 240
   aaacaacatt cattcaacaa acattttctg agacactgac catgtgccca gtgcaccagg 300
   ctatggctga ggtacaaaga caatgagaac agtctctttt tggaggctaa ttagtactct 360
   catttctacc agcactttct ttggcctttt aaaccaacca tttttgtcca gacaagacca 420
   aaaaaaaaaa aaaaacaaaa acaaaaaaca aaacaaaaca aaaaacccaa aaaaacaaaa 480
   aacaaaacaa ctgggttgct ttagggaagc ccggcttgga gtggacaatg gtgtccaggc 540
   ccctgaaagg ggctgattgg attatgaggc aaatggaggc agaaggagca cgcaggagac 600
   aaatgcagtg ggcggagacc cgctctgagt taatcaggct gattgaaggg accttttctg 660
   tcttgccgag tggcccgggg gggctctcac tagtgcactg ctgtgtgcac atggagactg 720
   gagctaccct ggtccctctc tggcaactgt tcattcattt gatgctcaca actccctgtc 780
   ctgacccagg gccggcctgc catcacgcag ggaagcagag gtcgccaagc ggtttccgac 840
   ggggcccccc cacataacgt tagcctcgcg gtctcttcgg ttttagcagc agtggacatg 900
   aacctgtttc cagcctgcat cctccagttc ctctcgggac tcctggctcc actgtgctca 960
   cctggagccc cgccagcccg cctccccctg tcctccttcc tcgccactcc agtgtctgtc 1020
   tttccttcac agccccggct tctcgaggcc tgtctacact cgctgctttc cttcctcacc 1080
   tccaatttcc cctccaaccc actgcttcct gactcgctct tctccatcga acggctctcg 1140
   ctcaggcctg tcgcgtcttc ctgtgccatt tgctgttgtg ctcaggttcc acgagctgcc 1200
   catctccaca gaagagcagc tggcattgcc caccggccat gccggctgaa gaaaagaaga 1260
   ctgaggaccc caggatgccc agcgcgagga ccccaggaag cccagcgcga ggactccacc 1320
   cacgacgccc agcgcgagga cttcacgcac aacacgttgc aaccctcctg gattaggcca 1380
   aagccatcat ctggaatcct gcgtgggacc ctggacacac ggacacagac acctgccccc 1440
   aggaccctcc aactgtaaat cgattggaag cgcccttgtg agctggggcc ttcaaagggc 1500
   tgaggagaaa atgcaggccg agagaccagc ttctcaaata ggcttcaagc ctccatgacc 1560
   tgagctcacc ccctggaaat gtctggaaaa cataatgggc aggttttctg tcttcaaagt 1620
   ttccatcaaa acctcttcaa gttctttatt gtttaggact gagacacttg gggacagtaa 1680
   tggggacttt cttttgtatc tgtcttgaaa ggccaaaata tttttatatt gctgtagaca 1740
   aagccaccta tttacaaatg gactcttgtt ccgtcgtttc ccaccaggaa gaccgtacta 1800
   tgtttgtgtc tctatgtatt ctggggtctt gaaacaggtt tctcatgggg atggccattc 1860
   actagagccc agaggggcag aaggggaagt gtctaccctg ccaagggggt ctggggaagg 1920
   aggggggcta gtctcagatt tgctccttgt atgtctaggg taaaccctgg gagaatctgg 1980
   tggtaaaaga acatatgctg tgtttgcccc acatctgcca tttgtgtgtg tttctgggct 2040
   acgggtttgc acactttggt ggttaacatg gtgggaggaa gtaagaaggg ccaagagggc 2100
   acgggtgctc tggttttcct gctgggaggc tgtttcactg gtgtgtgtta catcacataa 2160
   tgcatgttta gctgggtgag tgacaacagt atgaagggac agagaaacaa ataggtgtag 2220
   aaagagagtc acagagcaca cagtcaccat tctgtgccca ggcgttcatt aaatgttcaa 2280
   tagaatgctg agctctgata gacagagcgg gccatggaca cactggtatt gggtcatcat 2340
   ggacctaagg agttttttta attgaagaag gaatcttaca taacagaaga aagaataaga 2400
   agagctgtca aaacgagctc tgaggttttt gaagcccctt aatataaaca gacagacaat 2460
   ggcgtgatca ggaaagggag aaaacaaaca aacaaacaaa caagatgctt acagaaatac 2520
   gagaggacgt gtgagcatgc aaagagaacc cagctaggtt agtatcgggg tgctggtgga 2580
   tcgttaatcc tgtctgggta ccccagctcc aaggggatga gaggcagcaa gtgagaggac 2640
   acataggaaa ctgtaacaag acagaggcga gcagatgggg agcttcagcg gagtgttaag 2700
   ctaggcttcg caatctgacc acttaactct cccagcaaag aaaagaaagg cttaaatact 2760
   aaaaagaagg aaagctggaa gaaaggatgg tagatgtgaa aggcatagag gatagaaagc 2820
   aatagggagt ggagtgcagg tgctgtggag agacgcttgc aggtgactca attcaccagc 2880
   ctgtgcagct gagaaagaca gccagtgaca aaggagaaga agaaaggtaa caatacactc 2940
   acaaataggt agatgaatgt agctggaaga cggggcctgg ctagggacgg gatgagaggc 3000
   tgtgtcagca gactggttaa ttcccatctg tgcttctgag atggacaatg acccacccga 3060
   ccaagagacc tttcaggcat caatatgtca gggagggctt caatggggca atggaggcga 3120
   ggcccattaa tagcagatta tgatgtatcc aattaggtgc acctgccctc tggaagtgca 3180
   gaaaccagta ttgaaatgga aagaaagact gaatggaagt ccgagagtac aaatgaatgg 3240
   atgggaaaaa agatttgcat tcgctcttag ggggagggtg ctgctgagcc ccagggtcat 3300
   ctgatctccc ggagaataaa agggcgaggg agtgatagag agtaacaata agatggagaa 3360
   gaaatgaaaa ggagtacaga gagaataagt ggggacaatg aagtctagga gcgcggcttg 3420
   tttaacacaa gtcatccata ttaattcaaa attgaggccc tgggatggga tgggctgtgt 3480
   ccaagggatg aagctgttgc ctagcatgtg ccaagttctg ggttccatct ccgcctggca 3540
   agacaaacac aggaaacaaa attatagccc agaggttgta aatgttgttt ttgatggttt 3600
   tgggacaaag agctagatcg gttggaagat agagaaatag gaagaggtgg gaaagagtta 3660
   gatgagcaca agaggaagca gcagttctta agataagtgt ttaaatatgg tgaatgcgta 3720
   gctcttgggt gtgtcaagat gaaaataacg aaaagaaaca agtttaggga tgacggcaca 3780
   gggatgagcg gaagcggtga ctttagttaa tcaccgtggc caactcaagc cctgtcaggg 3840
   atttaaaaaa aggtacaaag gcgataaagg aagacacatg caatacggaa gagagagaca 3900
   ggacagacag acacagcgaa aaggctgagg acatgcctga gagggtcgcg aagggatgag 3960
   agagagaaca gcgagaattc tgcttaattc aaaaccgggc tcctgagata gctaaatatc 4020
   tcctgagccc aaacaccagc acaggagatt aaataacaaa agagctgcag gcagacccaa 4080
   gtggagaagg cttgggaccc tcagatcagg cttcaactaa atctgtgcac ctgaggtcca 4140
   taaataaaag aataaatatt gaaataaagg agtgacagaa aggatgaatg agcacacatg 4200
   actgaattag aaatggaaat ggctagagca gccagtaagg agtggcccat gggacttctg 4260
   ctcatcttat tctgggcacc tgaggtccct tcgtgagaag atgcagggag aagaggggaa 4320
   gggaaaggca tgagagcaaa tgaaagagag gcgaggagag gaaatgagaa agtggcagag 4380
   aggggaagcg atcggtgggt gcctcggaag aggaatgtcc acgaggatta ataacgaggc 4440
   tctcattaat cctaaagatc gatgaggtaa tccagaccca ggttcttaag gggaaggaga 4500
   atggagagaa ttaaaaaaaa aagacgcaaa aaaaggagag aaagataata aaacaagcaa 4560
   catgatgaat gggtaattaa gagaggctgt ctggacagaa atagtaatga gttcccatgc 4620
   atagtcctta aatagataaa tacattatac ctttcaaaga atgcgtgaaa aatcgaagaa 4680
   caccatggcc cccaaacaag gctgcacctt ccattgctgt aataaaatac atgcagcgga 4740
   gagccataaa taacggaaaa tacttaaatg taaaaggtga ccagacagag ggagaagatt 4800
   gcaagcccag atacacgagg gagggaggcg atgtttgctt tgcatgtgta atacaattat 4860
   gttcacagga aattaataga tttaaaaaaa aaatccaaac taagactggg agaaaaaaca 4920
   aggaagagag ggaagcgaga tctgagagat ggggctgaga ctcccggggt aggctcagca 4980
   tggttttgac tattctgcac atccggactt agaaaagtga gtttgtcagg gcaggaagag 5040
   aaaggtgaaa tctggagcgg gcagagctgg gcagatggat agaaaaggaa tggaaactac 5100
   acaggttgtg tgtgtgtgtg ggggggggaa gtgggggctc taagatctga ggttaggctt 5160
   gtttaaagct gagccattgt tgtccccaga ggaatcaaat cttactgctt ttgtgaagga 5220
   agagaggatg gcaacgatat tgcaggaaaa gagggaaggg ggtaggcaag gaggcaggga 5280
   ggaactttca tgtgctattc gctgactggg attggtaata gatagctctt ggcccaccac 5340
   aagctgcttc cacacagcct aactgactgg gagcagctat ggatcaccat ggccatgttt 5400
   tgcttgcagg gggccatttc actgtctgtc acatcttcag tccatagaca catccctgca 5460
   cagtgccctt tccatagacg tggataggag ggaagggctc aggcctgggg ctgtggagat 5520
   gattgcatga atagggggcg ctatgcccag tgggtacctt gtctcctctc tgggaaagga 5580
   tctcttctga tcctgatggg aaggggctta gccagccaaa gggcagggat aggaggcttt 5640
   gtctgctgcc tgctggctac catcttgagt gtgacttaca ccagggtgca gacaaagtga 5700
   gcttgacctg gaagcctaga ggcccaagag gcccgggtga agcaagtatc tctgagatca 5760
   agcctaccct tcagctcaga gagtggaccc ctgcctaggc tcccacacat gatcctaggt 5820
   cccagatatg ccacccctgg agcccgtttc aaggcccatc ttccagaatg tttaaacctg 5880
   tgtatgtaac cagtaaaatg ggagtttgta ctgtgtattg aatctgtctt cttattggcc 5940
   tctgtgggct gctgggggag acctggagag ttgtttattc tagtgtcccc caccccctgg 6000
   gactctttgc ttttgcccag ttttctttct ctgttgtgga taagattttg tagagttgtg 6060
   tgttgtatgc ttgtgtttga ccattaaaaa aaaaaaaaaa gtcacagccc tccttgattt 6120
   ctcaattcct attgggggtg ctaaattcca aaccaaacca atccaaacca gattcatgtc 6180
   aagtgcccaa caggaggata atagcttggc tggatcaagg gagcatgtgc agacccaaga 6240
   acagagtgga cagatgggct ataccgcaag ggggctttga gagctgagtg caggctaagt 6300
   agagagaagg tatggaaggt tggggagggt ggggctgctg ctacctgagt tgctgagggt 6360
   ctgagacacc agcaaggagg aggggcccct ggaagaagcg ttttgagatg actgaagatg 6420
   gaagatgggc gctccctacg cttctagctt caaggttttt agtgtacgcc agccgcctcc 6480
   gatcgtggaa gacacagcct aaccttgtca gaagccacct tctgtgcccc cagcaccacg 6540
   tgtctgggcc acgtgagcaa cgccacgtgg gcctgacgtg gagctggggc cgcaggggtc 6600
   tgatggcctc ctgtttcaga atctggggcg tttatcatcc ttgggacaac tgctgacctg 6660
   gctccggaag attgtccaca caagtctgag tgacggagtg agatgagggg gcgggtggag 6720
   atgctcagaa gaaaacagcc tttgcatggg gttcatttcc agctgtttag agagtcagcc 6780
   aaggtgacaa ggtggggttg gatgcggaga agaatgctgg gactacatgt gggctacagc 6840
   tgtgaagtca gtgacatggg gcagcatggg gatgggtctc taggtgggcc ttgtctcaac 6900
   attcatgcca gaagattgga aggggtacca tgggttactg tgggtcagtg ggaccttgga 6960
   aggaacaagg gcttgggtag gtgactttgc catgtcacct tgggatctag ttgttttgag 7020
   tctttgactc ttaatctgat cttgaataac aaatgtctct agttctgggg ttctgagagt 7080
   ctgtggtagg attccctagg attcgtgtgg ggagggcggt ctgcatgtgc tcccaagttg 7140
   gtgtcccagc ccttctgcct gctcagggct gtcttagaca tatttgcttt tcctttcaac 7200
   atcataaaag aaggcaaatt ttgtcttttg attgacactc atatgcaaac cacaacatcc 7260
   tttctctgat aatactggga cctacagctc tcctcggctc cccccgctat ttgagcccac 7320
   ctttggggca ggtaggccaa aggccctggt gttttctgag gtgaagttgt taatgtaaag 7380
   atgtgtaact cagagcaggg ggaagaaaca caccctcaac tctgcttccc cgtgctccat 7440
   cttcctttct gccttccaaa ttcttatgaa caccgttttt ttttttaaat ttttttccat 7500
   ttaatgagca tccttttttt tttttttaaa gctccattgg tggtgcttac ttttctgttg 7560
   gtgttttgtg tctgttgcaa tgagcatctt gctgtccacg ttttcgttgc tccatggtct 7620
   ctctcacctg cttgagttcc attgaatggc tatgacataa tgatctaccc atcttactgt 7680
   tgctgatgca tgatcatctc ctgtcctgtt tttttttttt attttacatt accgatgaca 7740
   ccataactaa tggctttgca caaccagctt tcccagcatc cctagcgccc cctacatcta 7800
   gctcttccca tggttccttt cttttctcct tgcttccttc tgctagactg tgagcccact 7860
   cagggcaagg agcttgtgga actgcgctct gtatttcttg taggatgtct atgaatgttg 7920
   actgcgtgtg tgtgtgtgtg tgtgtatgtg tgtgtgtgtg tgtgtgtgtg tgtgcgtgtg 7980
   tgcgtgtgtg cacgtgtgcg tgtgtgcgtg tgtgcatgtg tgcgtgtgcg tgtgtgtgtg 8040
   tgtaagggtg gacaggtatg agtgatggag tgattgaaag attggcagac agatgagagg 8100
   ctagatgaat ggatgagtga atgaatgaat gaatgagtga gtgaattaat ggatgggtat 8160
   attctctggt tgagcttctt ggatgaaagg gtaatgtttg aatggctgtt ggttgattga 8220
   agaaagccta cagtatttta tcttgtctga taatgtatta gcatttaagc tgatgtatta 8280
   gtatttaagg tgtctgctga ctttcctcac ctctctgtta aaaaatgctt ttgtgtgtgt 8340
   aaaacttgtt ctaattacta atgaaggctg aatacttttc acccatgttt gttaacaaat 8400
   tgtatcttat cttctgtgaa ctgtttgtcc aagtccagtg gctcatggct ctttcaggct 8460
   cttggttgag ggtgtcctta gactaggcaa tggcctttca cagatatcta cagaatgatg 8520
   tgagaggctc agctgacttc tagctcggca ttagaattta cttaccctgg gaaacaggga 8580
   ggctgatatc ttgcaccagc aggtagaaaa cacttttagg cttgtgtctg ggttcaccat 8640
   gcatttctag cttgtgagcc ctgacctgtg ctctgtacct cttgtggtgt ggcagtcctc 8700
   caccttgtcc tcctgagtga tagactacat atatgcctct cccctgttct ggggtgtagt 8760
   ctctgaggcc atgtcttaga ggaactggct tcttagtgct ctgtctagac atccccttag 8820
   ctctagtctt cccatgcacc tcagtggcag gtgtggtctt aatttaggga atgaatgaga 8880
   aattcagttc aggactgatg tgaccctcag gttagttcta ggagcaaggc cattttatgg 8940
   tctcggctct tcccaacctc ctcacagggc agttatagcc atctgggtag ggtgtgtgtt 9000
   ccgggagagc tcatcctgcc atccagaact ccctcccaca ctctattata gcacttagcg 9060
   tgtctgcctg tgttcagccc tctcacccca tgcttatctg gacattgaag cttggaaagc 9120
   cagtggtgac ttcaactgac ttttaattca tccacccatc catctggcta tccatctagc 9180
   catctgtcag tcaatccatc catccatcca tccatccatt catccatcca tccatgcata 9240
   cacacattgg gcctccatca cttgacctgg tgctttgggc ctctctccct ctcctgaaag 9300
   caaacccctt caccttgggc aagctgtgtg ataccatggg ctgtggtcat gaagcaaggc 9360
   ccccattcac agcctcctcc tcctcctcct aggtcacggc tctgagcacg tcccagctgg 9420
   acccctatca ccacagcttt ttctccataa ggcttctcac acctcacacc ctcctcctgt 9480
   gatccaggag ggccagattc ccagagtgcc ctggggctgg cccttcccac catccgcgga 9540
   gctgcctccc caggcttcac actgcctggt gcatggtccc tgtcatgagc ttggccttca 9600
   cctttgcagc ttcctccacc agatggcagt ggttgtgact cgtgtgccca ttccttcgca 9660
   ccttcattca ttcattctct tacagccagc aagcatctct ttaaaaaaaa aaaaattagc 9720
   tagtgctata ctagagccgg ctcacaagca cccattgcac cactgtcctt tgaaagaaaa 9780
   gatggggaca tttgtaaaga agctgtgttt gtgtggccaa taagaggagc tccttgattt 9840
   caattcaaac ttcaggtctg agttctattg ccagcttgat tgctctctcc aattttgttc 9900
   ctcacaaaac taggttgatt agtaatcagc aaactgtgtt ccctgggtgc tcagaaatca 9960
   tagcccagat ccttaagagg cccaaatggg gagtggacta agcacgaagc agctgcctct 10020
   ggctgcgagc aggctttcaa ggctgtgaga cactctgttc ttctatgagg atgatctgag 10080
   agccgttctg gccactttgg agctcagcta atgcttggct cctttttaga gaaaattgca 10140
   ggagatggat gctcgtacaa gtgacaagga tcatgtccca atgtcctttc tgaataatcc 10200
   attcgcagac ccaggcgagc aaggcacatg gcactttgca aacccctgtg tgtttcagga 10260
   ggacccatgc taggggaggc ctcctttcag ctttctgctg tgaaggggaa gagacgcagg 10320
   gctcttctga gttctcctcc cactagacgc agtaggggcg gcaaggccag aacctggagc 10380
   tggacaaatt gtgtgatgga tcttgcctca gggctgttgt gaggattggt ataactggac 10440
   acttctgact gtgaccctaa aaggcagtgt atgtgtccct gggggtgtgc cagagcattg 10500
   gaaaccctag ccctggagtg ggggtggcct tgcttgggta taatggggaa tactttgtat 10560
   ctgttaaatt tgtatatttt cctatagaga ctttaacacc acgggatagg ggactcttta 10620
   gatagtacct gtgccctccg cccccctcgg acacatccac acctacctct aggagaggtt 10680
   ggggttgtct ttagctctct gactgaggac caagcctctg actcagaact gtatatggca 10740
   cctagttaca tccctttcca aaaggctctt cccaggggag cactcggccc gatctggcag 10800
   accccattgt ccctttccca atgtcctctc tcccactaac agcatcaggc caaactgcct 10860
   gagatctggt gcctaccaca gtgcctggcc aggggtaggg cttcagtgac cttctgttgt 10920
   atttgtgtag gtagatgagt agctaacaat tgtaacaggt cctaggggca gatgtgtatg 10980
   gtctcattca gtggtttgta atggagaatg tatctgaacc catatcaagc catctctctt 11040
   ccttaacatg ttaagcagcc actgctgtcc tgggttgttt tgcaccttgc cttgttgtct 11100
   ttcatgctaa gtaaaaaaaa gtcctgcttt ctgctgagag tctgtttctt ctggaactca 11160
   cctaggtgtg atctggcagg gctgctaagc tgtgtctctg cccctctgac ccgtagtctt 11220
   cttctctctc aggtgtgcgt cactggcaag cctacctcac agggttgttg tgagggctaa 11280
   gggctgatcg gctgtaacag tgtgatgtcc atgctggctg tcaccaggga tggtgccatg 11340
   atggggttgg cttgaaatca caacctatac cgttattagt ggtcactgaa taaagagtct 11400
   cactgtggac actgaggttg taaaaaaaaa aaaaaaaaa 11439
<210> 271
   <211> 1317
   <212> DNA
   <213> Mus musculus
<400> 271
   ctcagaggat ctgtcaaagt accttagatt tgccctaatg gacataagca gcagtgggcg     60
   cagaaacctt gctctgaagc ctctctggtt ccaacatctg cggaagagtg cttgtgtgtc    120
   accttcagct ggcatctcca taacaccaaa attgaagtgt gagaagaaga agacccaatg    180
   cccggggaga agtacggtga gcctgtcatt attcagagag gctagatcct ctgtgttgag    240
   aaggatcatg atgggctcct cggtgttctc caggtagcgg caccacacca tgaaggcagc    300
   ccggattgga aggatcctca tctccactcg agggtactcc acctccattg tagagagggg    360
   tcttgaatag aaagcacagg tagatttctt gccagtttcg tcgtcggttt ggaccaggga    420
   ggcagacagg aatgacccag tgatgtctgt ttccaagtag aatgggttct gaggcttagg    480
   gtgatagaga acgggcgact tgcggaaagc cctcttcagg gattccaagg cctcctgctc    540
   ctcttctccc cagtagtagg gctctgaact cagcagttgt ctcactaggg gtgctgcgat    600
   gacagcgaag ttctccacga agtggcgata gggatagaca aggtcaatca cactttgaag    660
   acacctcctg ctgccaggga cagggcaccc cacgatgagg ttcataaggt tcttgttcag    720
   tttcacccct ttgggggata tgttgaagcc caagatttca gcggtctggc gatggaactg    780
   agttttgtcc agtgaacagt agatgttgtg ataccgaaag cggaccagga cttggcggac    840
   atgctgggag tgttcctcct ggctcattga gtagaccagg acctctctgc catggcaaat    900
   cacaaacaac cctaggatgt cttttaggat gaagtgaaca atgttgttcc cttcgtcaga    960
   gtatgagttc attgtgaagg gccggtagca tctcatctgg tgaaggccga aaccgaacga   1020
   tgctctccaa gtgtcttccg tgtgtgtgac cgtgtgcctc atctcacttt ccttaatccc   1080
   aagcagctcc agttttgtga accatgccgc tccatgtagc tggtcgaata gctctggtac   1140
   catctgcgtg tagtcttgtc tgtcagtcag catgtcatac agtatccagt attcctcttg   1200
   ctccctggct ttctcttgct gcctggcttt ctcttgcatc ctggcgctca caggttccaa 1260
   agtctctcgg gccccagact cgtaaaagac gccgctttga tcactgtctc cagcctg 1317
<210> 272
   <211> 3584
   <212> DNA
   <213> Mus musculus
<400> 272
   tcactgcccc tcagggggca gggggctggt gattcctgag ggagccctgc ccccttccag 60
   ggtgaatttt cctaagtcac catggagaac cagcagccca gaatcagtgc cctgccaggc 120
   agaattgctt ggccataaca aagatccatt gcctttctga tgacacccag cacctcggat 180
   tctgtataca cgggaggtga tttaagcatc ttactgccac cctgaaatct ggggtccttt 240
   atcctggcag catctagacc cgtaacgccc acttcagaat gggagggagg atgtctgctg 300
   ccctgtcgtc tccacacccg aggaatgtcc gtgtgtgtgt gtgtgtggtg tgcacagcgg 360
   gctgcacaca gcgggctcca cggtgctcga atggaggccc taatgtgaat ggagactagc 420
   ctgctggtag agaccttggc agtgaccgct gtgggcaatg agccatgggc caacaatatt 480
   caatgctgtg tacctgatgg atggttactg tctgagactg aggctttttg ggccaggagt 540
   aaaaggactt ggtttgtact tgattgcgtc ctgtggatgc ttgcctacca actgactcat 600
   cctgacgtgc caaaggtcct ggctttcccc tgtgtgggta agggaagaag agagttctaa 660
   tgaagactat agcagactga attactctct gagatgagga ctttgctatt gccattgcag 720
   aaccactggt ggaagttgtt gttaaacagt gggtcttgca tgcaagctct acagttatgc 780
   acagtatttc catgacatcc caagccatga ggttcatgct gccatatccc cctgtttaac 840
   aagtgtggaa actgataatg gactcacaat tgctgatgta taggttctaa tgttttctat 900
   tgtggacacg ggacacccag actgagaacc aatggctcca gtctgattta aacatgggtc 960
   cgtgctcaca cactcaatac ctaccctctg tgctcacaag tcctgtgtga gtaagactcc 1020
   aaattattct gtcttgtttt actcaatggt atgctctacc ttattataga gtataccatg 1080
   tattgagaca agaatgccag tcattctgag aaacaaaatg caaccattag ttgtcatttt 1140
   aattagtgac gaatagttgt gtcaatcatc aaacatgctt gtataattgg atgagaagag 1200
   gtgtaacacg ggccaatgat gacgaggttt cgggattata agtcacggac gatgactacg 1260
   tcactagagg tctgaggtcc atagcagaag atgcctcatg tgaccctctc atgcaggggt 1320
   tgcatgtcag gcatgtgcat acagtagata ttagccatgc actctcacca tttcatagta 1380
   gatcacagaa tcaagataca aatctccatg cacggaaagg atttaaggca ttgatcaatc 1440
   ctgaacataa atatttggcc atcaatgata tttacgcaca cactgaaaaa tctattcaga 1500
   atgacttaca caaaggttta tataaccttt atcaaaggta atttatatag taagattcat 1560
   ggctgaagtc ttcatgatgg tcaaggcctt aatttttaaa attatcaatt tttttctttt 1620
   cttttttttt aacttgaatt tttttttaat gaaaactatc atgacaattt gtctcttaaa 1680
   tgaaaggaga aatgtaaaac taaatggaca aatatcaacc tttttattat ctttgctgtt 1740
   gtgccctccc tggatgggtg ctggcacatg atgtcttgcc tgctggtccg tggttgcact 1800
   ctcaccatgt ggatggagct cggcagctga aaatgcattc ttccaccaag atgatttaca 1860
   cagcctagct ggatcaggag ccccctaata agctgcttca gattgtaata tagacacagt 1920
   aatgtatgcc tgcaggatga gtcccacctt cttcattagt cttgaggttt gagattccct 1980
   gtttgtgtct gcaggattca gatagtaatg gatatcctgc aagtcggctt cgtagagtct 2040
   cccttgaaag tggacctcat gatctagcct ttatgtccgt ggattgacac tgtgatggat 2100
   tccccccaag ttcatttaga tattctctct tattcataaa tgtcacggtc agctctgttc 2160
   ttatagatac cagaatggat acccctaagc tgatgtatat acacccttcc tcggttgtga 2220
   gattcctgct ctggtggttg tagtcataag atatacatcg ttgcgtacac cttggttgtg 2280
   ggcacgcaca ctactgttga attaccacct tgtccaagtg gatggatgcc ctcaatggga 2340
   ttggcacttt ctccattgct cctgcactct gttatcagca cttcgggttc ataatgtaca 2400
   cacagcaatg gattttctgc agctgatttt ctatattctg ttttcttcgg gtaagcagaa 2460
   acagatgcct tcctcactgg tcaccaggtt caaggtccct catatgtgtc aggtgcatag 2520
   tcaccaacaa aggccaactt cacatcaatg ggtggatcgt acctcggcct aagctccagg 2580
   attgattgtg ctgttagagt cacagctaca accggctcag ggaacccagt ggaagaggag 2640
   gtggagtgtg agagccaatt ggaatggaag acagtgagga aacaaggcct tcgagacaca 2700
   agaggactgc tgagctcaca aagactgagg cagcaccctc aggacctgca caggtctaag 2760
   cttgatgcga tctgagtact gagagggcaa ctggacacaa ttctatattg ataacccaga 2820
   agctttctcc agttgataaa ctctttcaaa ggaaaaactt gttttctacc atggagtgac 2880
   actggatata gaaactaccg ttaacagcag actccatggc tcagacttcc aatacaatat 2940
   gaactcagtt gcaatttttt gcaaggtttt tcgactcata gttctttgtc tgggtacttt 3000
   ttcaccctac agagaatttc tgtatatatt atcgtttctg attctagtgt tttgaatttt 3060
   cgctgggtct aaatatctct gtctcagtgt cagtatgtgt ttctcatgat ttttctttgc 3120
   ttctcttttt cctattcttt gcttgtattc cctggtttgt gtttgagtgt ctgggaactc 3180
   tgagggtctg gttggtgata ttgttgttct tcctatgggg ttgaaaaccc cttcagctcc 3240
   ttcagccttt cactaagtcc tccattatta tgattcctat gctcagtcca gtggttggat 3300
   gtgagccccc ccccccctct gcatttgtca ggctccggca gagcctctaa ggagatagct 3360
   atattaggct cttgtcagct ttgtttgttt ttattctgtc ttctctatac tgtttatttt 3420
   agataactgt gtttattcta atgtatgaat gaataaatgg gtgtgtgttt gggtgggtgg 3480
   ggaattgggg agggtggggg tggatgtggg aggggaaact gtaacacaaa tacattgtat 3540
   aaaagtacat tttaataaat gtatttcttc tatgactaaa aaaa 3584
<210> 273
   <211> 1315
   <212> DNA
   <213> Mus musculus
<400> 273
   ctctgcctca gaggtcttct caagccacta ctgttcccaa ggatgcctaa ttgcaatgcc      60
   agcttctggg gtgaacatgg acccactgtc atcgggcaag gtctaggatg gacatggaga     120
   cccaccatct gggagattcc agagtggacg tgaacccacc atcatcagag agcttcatgg     180
   agtattcaga agctggcggg tgtcacctgg aggatgtgac tgggtaccag tgccagagca     240
   cgtgcccact gagcagtctt cttcactttc ctcgatcctc atgaaacatt ccgggatctt     300
   tacctcaaag gactccagag gctacatcat tgtcttggaa ctggtcagtg gctggagctc     360
   tctccaggac aagtgcttgg aggaagaacc tggacaagct tccctcccca tactgttgac     420
   tggatcttgt agctaccgtt agccaccatc atcgccattg tgtgcctctt aggagcattt     480
   ccaggaggtg atgccagctg gggaaagagc ctctgtccca cagccgtcgg tgcccgctgc     540
   agcctgcgcc caggatgtca cagcctcagc tcaacacctc tgctggacag cttcaggtgg     600
   tgattccagc tccagggttg ctgcggtcaa cactgggtac ttgaggagag gttgtctgtg     660
   atgagttcgc tttattaatg acgaatataa cacagatggc ctgttttcaa taccacctgc     720
   cactccggtc actcggcagt acataccagg tgtcacacca ctgcccggca ggtgacaacg     780
   ctgaattggt cctcaggagc ggatgttcaa ggaaccctgc ctatgcacca tgctagctgc     840
   cagtcctagg ttgggacatt ccactgggga catccatggg agagctatgg ccattaacaa     900
   gtcctgagcc ctgacctgaa gccatcagtg gcctagctgc tctacctgac tttgtggcct     960
   cccttcctgg atctctcgct tacgacaacc gacaacaaag atgtttgact ccagaagatg    1020
   ctccccgcgg cccacctgag aggactgtcc acaccccacc ccactgcgcc ctgtgccatc    1080
   tacctctgag tccccaactt ctcacccatg tttcaactcc caccccagag ccttgtattc    1140
   cagatctgag cagcccttgc ttaggaacct gaggtggggg gtgggtaggc agacccaggg    1200
   gactgagcag ggaggggaag gaggggtttc tgctcttggc tttcaaagtt ctcagtgtgt    1260
   gtagcccttt gaataataaa catatgtcat ataatgtaaa aaaaaaaaaa aaaaa         1315
<210> 274
   <211> 1595
   <212> DNA
   <213> Homo sapiens
<400> 274
   agcccctagc gcagacggcg gagagcagag agggagcgcg ccttggctcg ctggccttgg 60
   cggcggctcc tcaggagagc tggggcgccc acgagaggat ccctcacccg ggtctctcct 120
   cagggatgac atcatccgtc cacctccttg tcttcaagga ccacctcctc tccatgctga 180
   gctgctgcca aggggcctgc tgcccatcta cacctcacga gggcactagg agcacggttt 240
   cctggatccc accaacatac aaagcagcca ctcactgacc cccaggacca ggatggcaaa 300
   ggatgaagag gaccggaact gaccagccag ctgtccctct tacctaaaga cttaaaccaa 360
   tgccctagtg agggggcatt gggcattaag ccctgacctt tgctatgctc atactttgac 420
   tctatgagta ctttcctata agtctttgct tgtgttcacc tgctagcaaa ctggagtgtt 480
   tccctcccca agggggtgtc agtctttgtc gactgactct gtcatcaccc ttatgatgtc 540
   ctgaatggaa ggatcccttt gggaaattct caggaggggg acctgggcca agggcttggc 600
   cagcatcctg ctggcaactc caaggccctg ggtgggcttc tggaatgagc atgctactga 660
   atcaccaaag gcacgcccga cctctctgaa gatcttccta tccttttctg ggggaatggg 720
   gtcgatgaga gcaacctcct agggttgttg tgagaattaa atgagataaa agaggcctca 780
   ggcaggatct ggcatagagg aggtgatcag caaatgtttg ttgaaaaggt ttgacaggtc 840
   agtcccttcc cacccctctt gcttgtctta cttgtcttat ttattctcca acagcactcc 900
   aggcagccct tgtccacggg ctctccttgc atcagccaag cttcttgaaa ggcctgtcta 960
   cacttgctgt cttccttcct cacctccaat ttcctcttca acccactgct tcctgactcg 1020
   ctctactccg tggaagcacg ctcacaaagg cacgtgggcc gtggcccggc tgggtcggct 1080
   gaagaactgc ggatggaagc tgcggaagag gccctgatgg ggcccaccat cccggaccca 1140
   agtcttcttc ctggcgggcc tctcgtctcc ttcctggttt gggcggaagc catcacctgg 1200
   atgcctacgt gggaagggac ctcgaatgtg ggaccccagc ccctctccag ctcgaaatcc 1260
   ctccacagcc acggggacac cctgcaccta ttcccacggg acaggctgga cccagagact 1320
   ctggacccgg ggcctcccct tgagtagaga cccgccctct gactgatgga cgccgctgac 1380
   ctggggtcag acccgtgggc tggacccctg cccaccccgc aggaaccctg aggcctaggg 1440
   gagctgttga gccttcagtg tctgcatgtg ggaagtgggc tccttcacct acctcacagg 1500
   gctgttgtga ggggcgctgt gatgcggttc caaagcacag ggcttggcgc accccactgt 1560
   gctctcaata aatgtgtttc ctgtcttaac aaaaa 1595
<210> 275
   <211> 498
   <212> DNA
   <213> Homo sapiens
<400> 275
   ggtctgaaaa atgatattca ttgtcctaat gtgtaaattt cgacaatttg caaatttgta 60
   gattctttag aatagaacta actcaagccc ttcattctgc agctgaggct cactgccccc 120
   agtgggcagt gggtccaggg ggtttctgag gacagggcat gacccagccc tgctgccccc 180
   aagatggcac ctggcttgga ggggtgaggg gccctgttag tctgactttg aagaagacca 240
   gccttccaga ctcgcttggt gccctgacag gagccctggg ctcccccagt gttgcctggg 300
   tctgactttg cctcagtgaa aactgcctcg aattctttct tgcaccgatg ggcagatggg 360
   cagtgtcgga ggatcgtgtc atctgtcccg tggcgctggt tggcttggtc aagtcagtgt 420
   tcaaactatc tcctgctctt tcaaggggat ctggggctct agaagattag aggacttgga 480
   gaggttagtg acttgctc 498

## Description

### BACKGROUND OF THE INVENTION

### Technical Field:

The present invention relates to a method for screening induced pluripotent stem cells. More specifically, the present invention relates to miRNA or genes that are expressed in induced pluripotent stem cells, or a method for selecting induced pluripotent stem cells having functions equivalent to those of embryonic stem cells.

### Background Art:

In recent years, mouse and human induced pluripotent stem cells (iPS cells) have been successively established. Yamanaka et al., have induced iPS cells by introducing Oct3/4, Sox2, Klf4, and c-Myc genes into mouse-derived fibroblasts so as to enable the forced expression of such genes (WO 2007/069666 A1 and Takahashi, K. and Yamanaka,

S., Cell, 126: 663-676 (2006)). Subsequently, it has been revealed that iPS cells can also be prepared using 3 of the above factors excluding the c-Myc gene (Nakagawa, M. et al., Nat. Biotethnol., 26: 101-106 (2008)). Furthermore, Yamanaka et al., have succeeded establishing iPS cells by introducing the 4 above genes into human skin-derived fibroblasts, similarly to the case involving mice (WO 2007/069666 A1 and Takahashi, K. et al., Cell, 131: 861-872 (2007)). Meanwhile, Thomson et al.,' s group has prepared human iPS cells using Nanog and Lin28 instead of Klf4 and c-Myc (WO 2008/118820 A2 and Yu, J. et al., Science, 318: 1917-1920 (2007)). The thus obtained iPS cells are prepared using cells from a patient to be treated, following which they can be differentiated into cells of different tissues. Thus, it is expected that iPS cells will be used as rejection-free grafting materials in the field of regenerative medicine. However, the thus established iPS cells exert almost the same appearance and expression status of undifferentiated specific genes as those of ES cells, but the involvement in the germ line may differ from the case of ES cells (Okita K. et al., Nature, 448: 313-317 (2007)). Pick et al. (2009), Stem Cells 27: 2686-2690, discloses the analysis of gene expression levels and methylation levels in such iPS cells.

Also, many clones can be obtained simultaneously with the use of iPS cells, but they do not always have identical functions.

Therefore, a method for selecting iPS cells that have unlimitedly high differentiation potency, as in the case of ES cells, from among many established iPS cells has been required. However, a method that involves confirming the presence of iPS cell-derived tissue in 2^{nd}-generation mice obtained by mating iPS cell-derived chimeric mice takes a great deal of time. Also, such confirmation using human iPS cells poses a major ethical problem. Hence, it is difficult to detect whether or not established iPS cells have differentiation potency that enables germline transmission.

### SUMMARY OF INVENTION

An object of the present invention is to provide an index for conveniently screening for an induced pluripotent stem cell(s) (iPS cell(s)) having unlimitedly high differentiation potency and being capable of germline transmission. The iPS cells can be induced from somatic cells of a subject, which is an animal, preferably a mammal including humans, mice, rats, pigs, cows, and the like.

The present inventors have confirmed microRNA (hereinafter, miRNA) expression using iPS cells having various backgrounds to achieve the above object. As a result, the present inventors have found that iPS cells capable of germline transmission and iPS cells incapable of germline transmission can be distinguished based on miRNA that is expressed in the Dlkl-Dio3 region as an imprinted region. Also, the present inventors have found that, among the expression levels of genes located within the same region as that of the above miRNA, a similar correlation exists with regard to the expression levels of genes that are expressed from maternally derived chromosomes. Thus, they have confirmed that such miRNA can be used as an index for screening for iPS cells in which germline transmission occurs. They have also found that iPS cells can be similarly screened for by confirming DNA methylation in a region that controls the expression of genes of the Dlkl-Dio3 region.

Based on the above results, the present inventors have found that iPS cells having unlimitedly high differentiation potency and being capable of germline transmission as in the case of ES cells can be selected by detecting miRNA or the gene of imprinted region or DNA methylation in imprinted region. Thus, they have completed the present invention.

The present invention is as follows.
1. A method of screening for an induced pluripotent stem cell(s) having unlimitedly high differentiation potency, comprising the following steps of:
   (1) measuring the expression level of gene(s) or miRNA located in an imprinted region in a subject induced pluripotent stem cell(s); and,
   (2) selecting the induced pluripotent stem cell(s) expressing the gene(s) or the miRNA at a higher level than that of a control cell(s),
   wherein the miRNA located in an imprinted region is selected from the group consisting of the pri-miRNAs shown in Tables 1 and 3 and the mature-miRNAs shown in Tables 2 and 4, wherein the gene(s) located in an imprinted region is/are selected from the group consisting of Gtl2/MEG3, Rtl1as/anti-Peg11, Rian/MEG8, and Mirg/Meg9, and wherein the control cell(s) is/are an iPS cell(s) or an embryonic stem cells (ES cells) known not to have unlimitedly high differentiation potency.

### BRIEF DESCRIPTION OF DRAWINGS

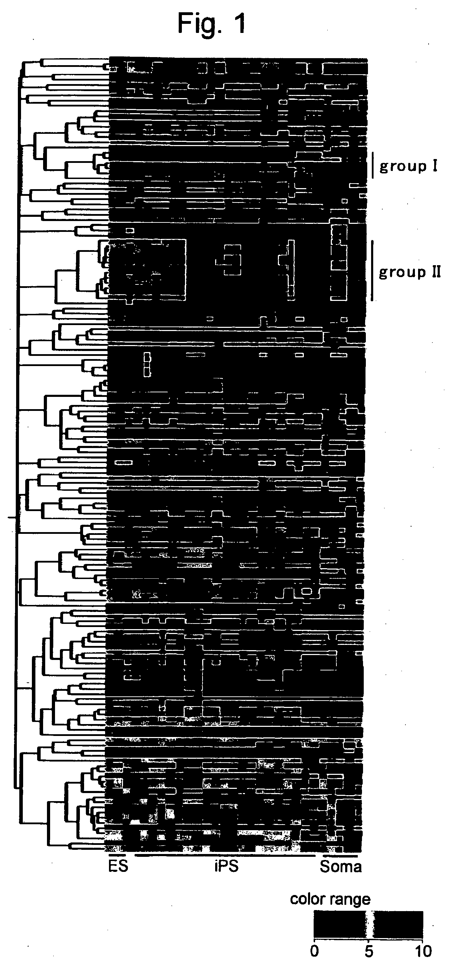

**Fig. 1** shows the results of hierarchical clustering of microarray data of miRNA expressed in ES cells, iPS cells, and somatic cells. Here, values within the color range are log2 values of detected signal intensity. Red indicates strong expression signals and blue indicates weak expression signals. Group I is a group specifically expressed in ES cells and iPS cells. Group II is a group expressed non-specifically among iPS cells.

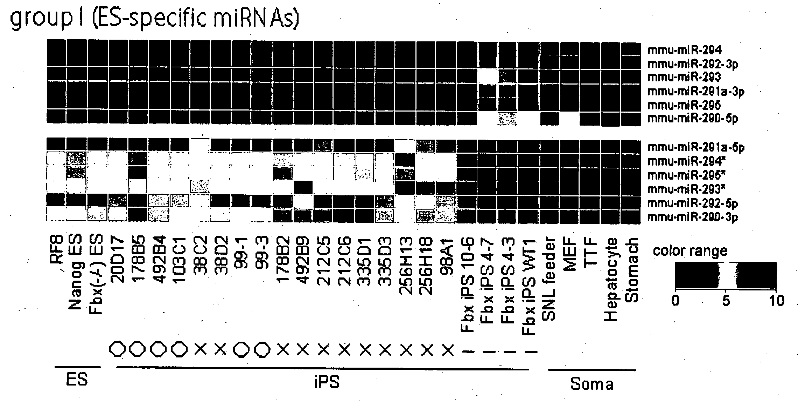

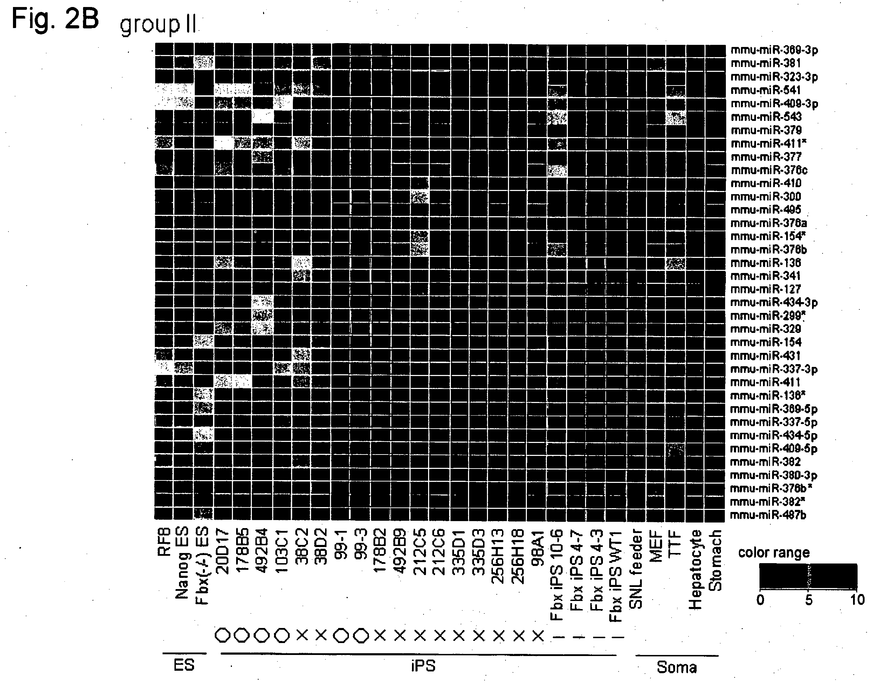

**Fig. 2** shows the results of detailed microarray analyses for miRNA (A) of Group I and miRNA (B) of Group II in ES cells, iPS cells, and somatic cells. The clone name of each cell is shown in the lower area and the ID names of miRNA are shown in the area on the right. Here, values in the color range are log2 values of detected signal intensity. Red indicates strong expression signals and blue indicates weak expression signals.

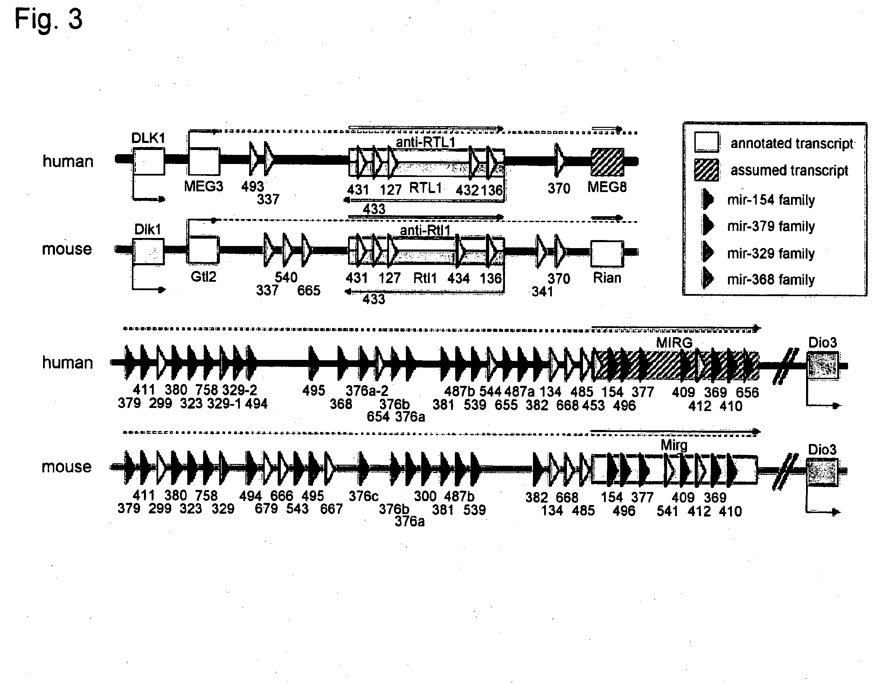

**Fig. 3** is a schematic diagram showing locations of miRNA and genes in human and mouse Dlk1-Dio3 regions.

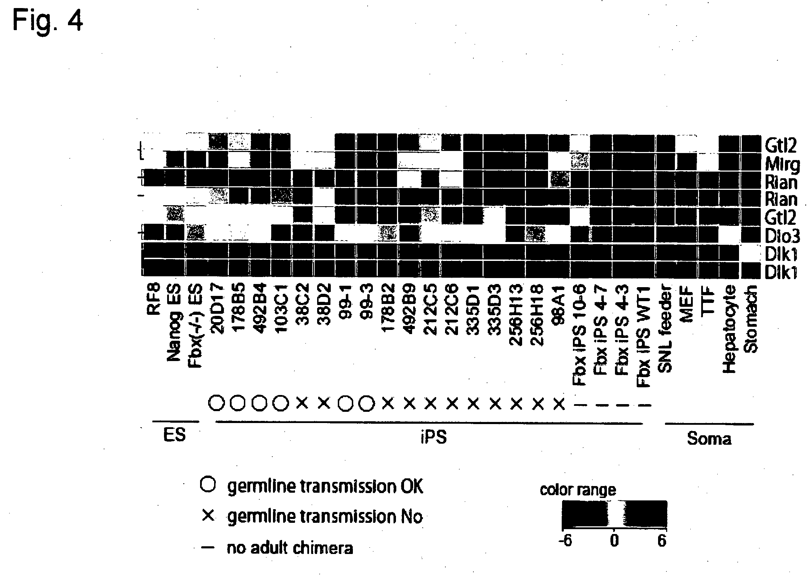

**Fig. 4** shows the results of microarray analyses by which the expression of genes located in the Dlk1-Dio3 region in ES cells, iPS cells, and somatic cells was examined. The clone name of each cell is shown in the lower area and gene names are shown in the right area. Results are normalized by the Quantile normalization method and expressed by signal intensity. Here, Red indicates strong expression signals and blue indicates weak expression signals.

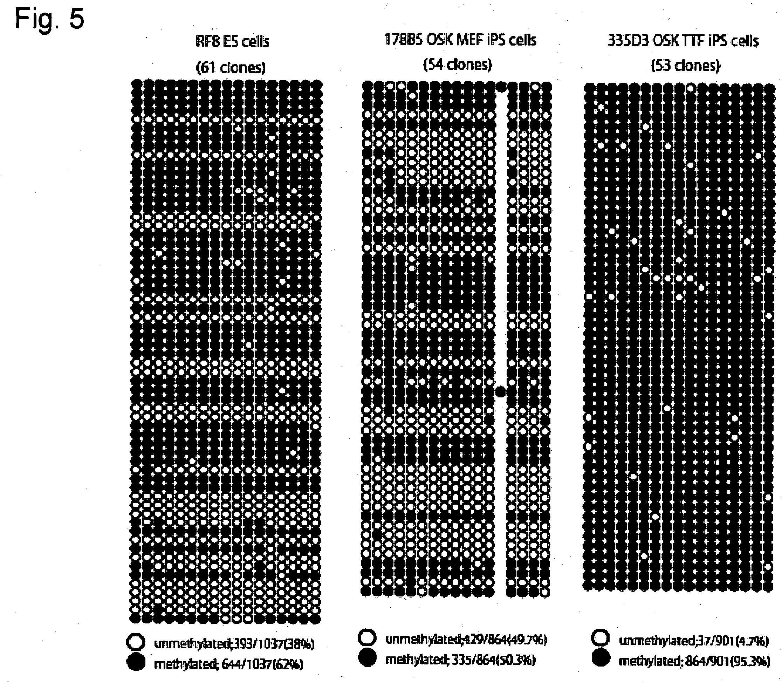

**Fig. 5** shows the results of measuring the methylation state of CG sequences at 17 positions in IG-DMR of ES cells (RF8) and iPS cells (178B5 and 335D3) by the Bisulfite method. A filled circle indicates that the CG sequence was methylated and an open circle indicates that the CG sequence was not methylated. The measurement results shown in Fig. 5 were: 61 clones for RF8, 54 clones for 178B5, and 53 clones for 335D3.

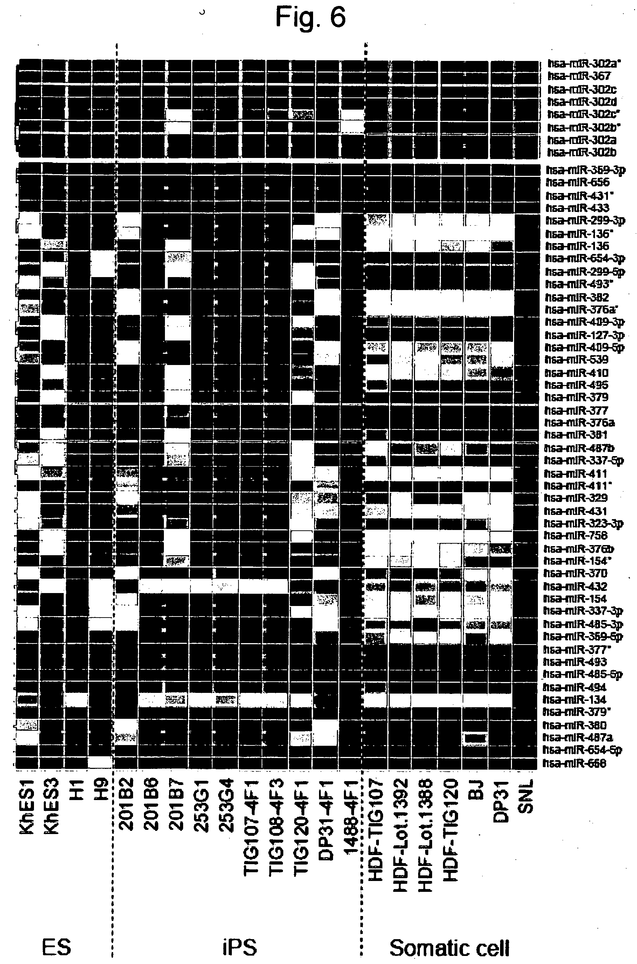

**Fig. 6** shows the results of microarray analyses by which the expression of miRNA located in the DLK1-DIO3 region in human ES cells, human iPS cells, and human somatic cells was examined. The clone name of each cell is shown in the lower area and miRNA names are shown in the right area. Results are normalized by the Quantile normalization method and expressed by signal intensity. Here, Red indicates strong expression signals and blue indicates weak expression signals.

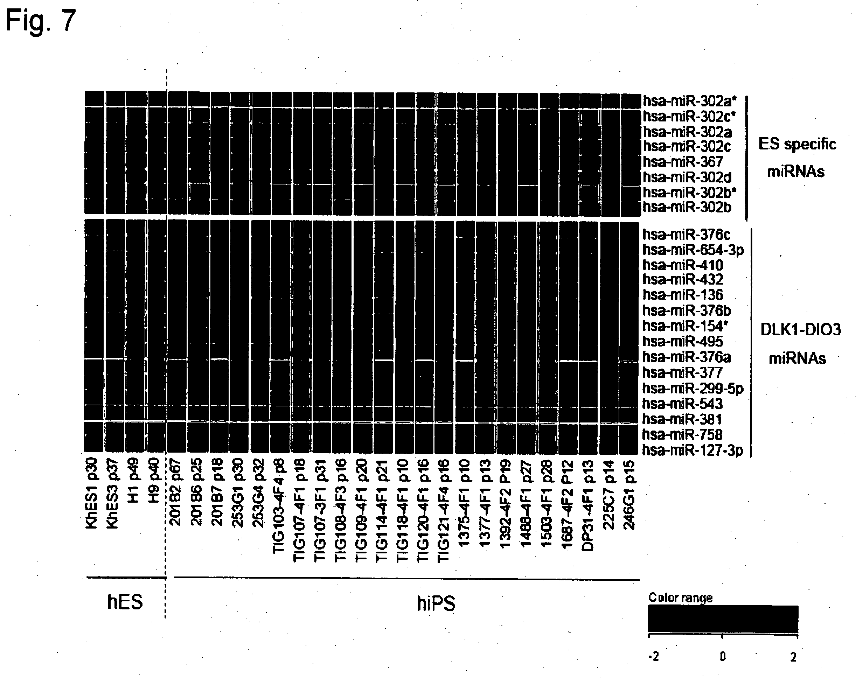

**Fig. 7** shows the results of microarray analyses by which the expression of miRNA located in the DLK1-DIO3 region in human ES cells and human iPS cells was examined. The clone name is shown in the lower area and miRNA names are shown in the right area. The number following clone name means passage number. Results are normalized by the Quantile normalization method and expressed by signal intensity. Here, Red indicates strong expression signals and green indicates weak expression signals.

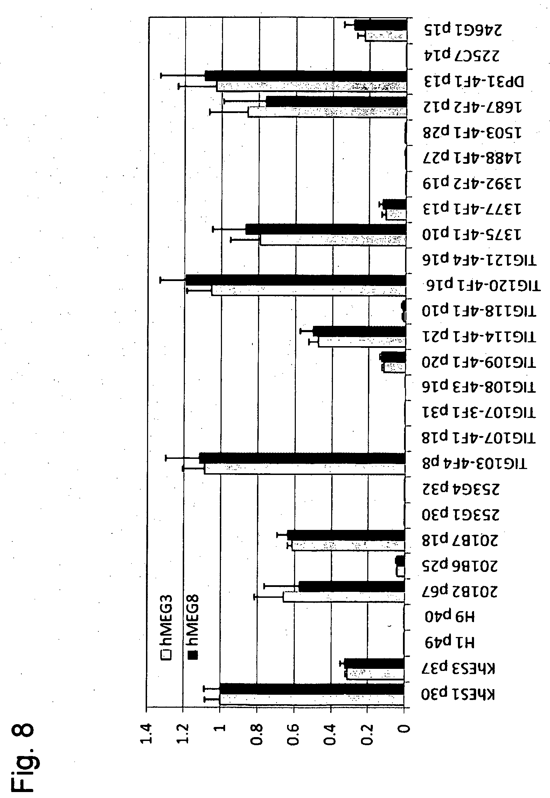

**Fig. 8** shows the results of expression level of MEG3 (gray-bar) and MEG8 (black-bar) in each cell line measuring with quantitative PCR. The clone name is shown in the lower area. The expression level of KhES1 is used as standard and each level is normalized with GAPDH expression level.

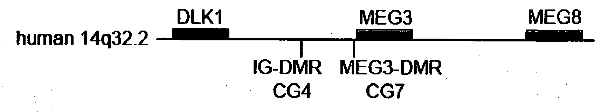

**Fig. 9** is a schematic diagram showing locations of IG-DMR CG4, MEG3-DMR CG7 and relating genes.

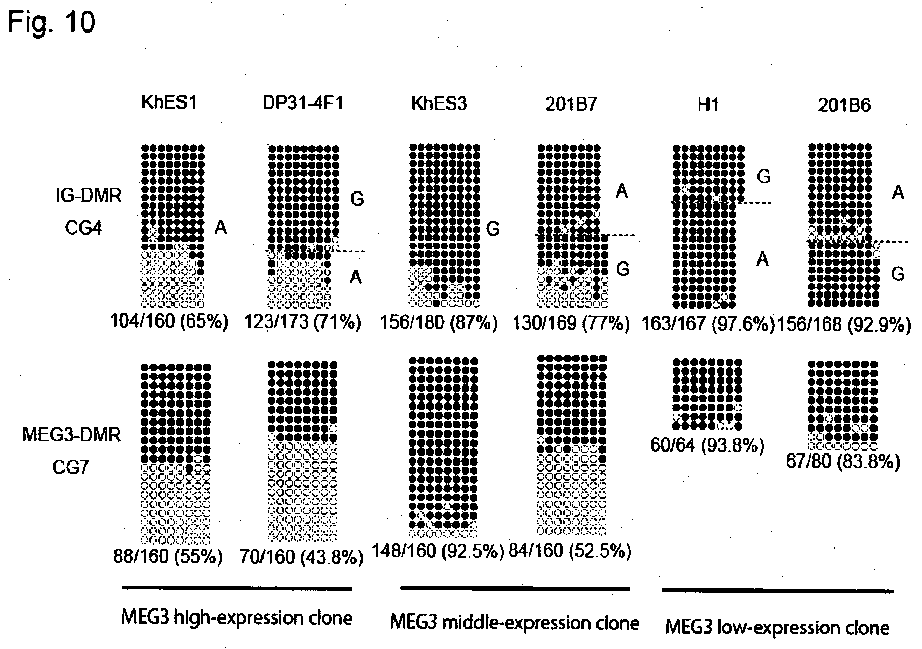

**Fig. 10** shows the results of measuring the methylation state of CG sequences in IG-DMR CG4 and MEG3-DMR CG7 of 3 clones of ES cells (KhES1, KhES3 and H1) and 3 clone of iPS cells (DP31-4F1, 201B7 and 201B6) by the Bisulfite method. There are 8 CG positions (indicating "A") and 9 CG positions (indicating "G"), because of SNP (A/G) in IG-DMR CG4 region. A filled circle indicates that the CG sequence was methylated and an open circle indicates that the CG sequence was not methylated.

### MODES FOR CARRYING OUT THE INVENTION

The present invention provides a method for screening for induced pluripotent stem cells (iPS cells) having unlimitedly high differentiation potency and being capable of germline transmission.

### Method for producing iPS cells

Induced pluripotent stem (iPS) cells can be prepared by introducing a specific nuclear reprogramming substance in the form of DNA or protein into somatic cells. iPS cells are somatic cell-derived artificial stem cells having properties almost equivalent to those of ES cells, such as pluripotency and proliferation potency via self-renewal (K. Takahashi and S. Yamanaka (2006) Cell, 126: 663-676; K. Takahashi et al. (2007) Cell, 131: 861-872; J. Yu et al. (2007) Science, 318: 1917-1920; M. Nakagawa et al. (2008) Nat. Biotechnol., 26: 101-106; international publication WO 2007/069666). A nuclear reprogramming substance may be a gene specifically expressed in ES cells, a gene playing an important role in maintenance of undifferentiation of ES cells, or a gene product thereof. Examples of such nuclear reprogramming substance include, but are not particularly limited to, Oct3/4, Klf4, Klf1, Klf2, Klf5, Sox2, Sox1, Sox3, So15, Sox17, Sox18, c-Myc, L-Myc, N-Myc, TERT, SV40 Large T antigen, HPV16 E6, HPV16 E7, Bmil, Lin28, Lin28b, Nanog, Esrrb, and Esrrg. These reprogramming substances may be used in combination upon establishment of iPS cells. Such combination may contain at least one, two, or three reprogramming substances above and preferably contains 4 reprogramming substances above.

The nucleotide sequence information of the mouse or human cDNA of each of the above nuclear reprogramming substances and the amino acid sequence information of a protein encoded by the cDNA can be obtained by referring to NCBI accession numbers described in WO 2007/069666. Also, the mouse and human cDNA sequence and amino acid sequence information of L-Myc, Lin28, Lin28b, Esrrb, and Esrrg can be each obtained by referring to the following NCBI accession numbers. Persons skilled in the art can prepare desired nuclear reprogramming substances by a conventional technique based on the cDNA sequence or amino acid sequence information.

| Gene name | Mouse | Human |
|---|---|---|
| L-Myc | NM_008506 | NM_001033081 |
| Lin28 | NM_145833 | NM_024674 |
| Lin28b | NM_001031772 | NM_001004317 |
| Esrrb | NM_011934 | NM_004452 |
| Esrrg | NM_011935 | NM_001438 |

These nuclear reprogramming substances may be introduced in the form of protein or mature mRNA into somatic cells by a technique such as lipofection, binding with a cell membrane-permeable peptide, or microinjection. Alternatively, they can also be introduced in the form of DNA into somatic cells by a technique such as a technique using a vector such as a virus, a plasmid, or an artificial chromosome, lipofection, a technique using a liposome, or microinjection. Examples of a viral vector include a retrovirus vector, a lentivirus vector (these are according to Cell, 126, pp.663-676, 2006; Cell, 131, pp.861-872, 2007; and Science, 318, pp. 1917-1920, 2007), an adenovirus vector (Science, 322, 945-949, 2008), an adeno-associated virus vector, and a Sendai virus vector (Proc Jpn Acad Ser B Phys Biol Sci. 85, 348-62, 2009). Also, examples of an artificial chromosome vector include a human artificial chromosome (HAC), a yeast artificial chromosome (YAC), and a bacterial artificial chromosome (BAC and PAC). As a plasmid, a plasmid for mammalian cells can be used (Science, 322: 949-953, 2008). A vector can contain regulatory sequences such as a promoter, an enhancer, a ribosome binding sequence, a terminator, and a polyadenylation site, so that a nuclear reprogramming substance can be expressed. A vector may further contain, if necessary, a selection marker sequence such as a drug resistant gene (e.g., a neomycin resistant gene, an ampicillin resistant gene, and a puromycin resistant gene), a thymidine kinase gene, and a diphtheria toxin gene, and a reporter gene sequence such as a green fluorescent protein (GFP), β glucuronidase (GUS), and FLAG. Also, in order to cleave both a gene encoding a nuclear reprogramming substance or a promoter and a gene encoding a nuclear reprogramming substance binding thereto after introduction into somatic cells, the above vector may have LoxP sequences located before and after the relevant portion. Furthermore, the above vector may also contain EBNA-1 and oriP, or Large T and SV40ori sequences so that they can be episomally present and replicated without incorporation into a chromosome.

Upon nuclear reprogramming, to improve the efficiency for inducing iPS cells, in addition to the above factors, histone deacetylase (HDAC) inhibitors [e.g., low-molecular weight inhibitors such as valproic acid (VPA) (Nat. Biotechnol., 26(7): 795-797 (2008)), trichostatin A, sodium butyrate, MC 1293, and M344, and nucleic acid expression inhibitors such as siRNA and shRNA against HDAC (e.g., HDAC1 siRNA Smartpool^{®} (Millipore) and HuSH 29mer shRNA Constructs against HDAC1 (OriGene))], DNA methyltransferase inhibitors (e.g., 5'-azacytidine) (Nat. Biotechnol., 26(7): 795-797 (2008)), G9a histone methyltransferase inhibitors [e.g., low-molecular-weight inhibitors such as BIX-01294 (Cell Stem Cell, 2: 525-528 (2008)) and nucleic acid expression inhibitors such as siRNA and shRNA against G9a (e.g., G9a siRNA (human) (Santa Cruz Biotechnology))], L-channel calcium agonists (e.g., Bayk8644) (Cell Stem Cell, 3, 568-574 (2008)), p53 inhibitors (e.g., siRNA and shRNA against p53) (Cell Stem Cell, 3, 475-479 (2008)), Wnt Signaling (e.g., soluble Wnt3a) (Cell Stem Cell, 3, 132-135 (2008)), cytokines such as LIF or bFGF, ALK5 inhibitors (e.g., SB431542) (Nat Methods, 6: 805-8 (2009)), mitogen-activated protein kinase signaling inhibitors, glycogen synthase kinase-3inhibitors (PloS Biology, 6(10), 2237-2247 (2008)), miRNA such as miR-291-3p, miR-294, and miR-295 (R. L. Judson et al., Nat. Biotech., 27: 459-461 (2009)), for example, can be used.

Examples of a culture medium for inducing iPS cells include, but are not limited to, (1) a DMEM, DMEM/F12, or DME medium containing 10-15% FBS (these media may further appropriately contain LIF, penicillin/streptomycin, puromycin, L-glutamine, nonessential amino acids, Beta-mercaptoethanol, and the like), (2) a medium for ES cell culture containing bFGF or SCF, such as a medium for mouse ES cell culture (e.g., TX-WES medium (Thromb-X)), and a medium for primate ES cell culture (e.g., a medium for primate (human &monkey) ES cells, ReproCELL, Kyoto, Japan).

An example of culture methods is as follows. Somatic cells are brought into contact with nuclear reprogramming substances (DNA or protein) on a DMEM or DMEM/F12 medium containing 10% FBS at 37°C in the presence of 5% CO₂ and are cultured for about 4 to 7 days. Subsequently, the cells are reseeded on feeder cells (e.g., mitomycin C-treated STO cells or SNL cells). About 10 days after contact between the somatic cells and the nuclear reprogramming factors, cells are cultured in a bFGF-containing medium for primate ES cell culture. About 30-45 days or more after the contact, iPS cell-like colonies can be formed. Cells may also be cultured under conditions in which the oxygen concentration is as low as 5%-10% in order to increase the efficiency for inducing iPS cells.

Alternatively, cells may be cultured using a DMEM medium containing 10% FBS (which may further appropriately contain LIF, penicillin/streptomycin, L-glutamine, nonessential amino acids, beta-mercaptoethanol, and the like) on feeder cells (e.g., mitomycin C-treated STO cells or SNL cells). After about 25-30 days or more, ES cell-like colonies can be formed.

During the above culture, medium exchange with fresh medium is preferably performed once a day from day 2 after the start of culture. In addition, the number of somatic cells to be used for nuclear reprogramming is not limited, but ranges from approximately 5 × 10³ to approximately 5 × 10⁶ cells per culture dish (100 cm²).

When a gene containing a drug resistant gene is used as a marker gene, cells expressing the marker gene can be selected by culturing the cells in a medium (selective medium) containing the relevant drug. Also, cells expressing the marker gene can be detected when the marker gene is a fluorescent protein gene, through observation with a fluorescence microscope, by adding a luminescent substrate in the case of a luminescent enzyme gene, or adding a chromogenic substrate in the case of a chromogenic enzyme gene.

The term "somatic cells" as used herein may refer to any cells other than germ cells from mammals (e.g., humans, mice, monkeys, pigs, and rats). Examples of such somatic cells include keratinizing epithelial cells (e.g., keratinizing epidermal cells), mucosal epithelial cells (e.g., epithelial cells of the surface layer of tongue), exocrine epithelial cells (e.g., mammary glandular cells), hormone-secreting cells (e.g., adrenal medullary cells), cells for metabolism and storage (e.g., hepatocytes), boundary-forming luminal epithelial cells (e.g., type I alveolar cells), luminal epithelial cells of internal tubules (e.g., vascular endothelial cells), ciliated cells having carrying capacity (e.g., airway epithelial cells), cells for secretion to extracellular matrix (e.g., fibroblasts), contractile cells (e.g., smooth muscle cells), cells of blood and immune system (e.g., T lymphocytes), cells involved in sensation (e.g., rod cells), autonomic nervous system neurons (e.g., cholinergic neurons), sense organ and peripheral neuron supporting cells (e.g., satellite cells), nerve cells and glial cells of the central nervous system (e.g., astroglial cells), chromocytes (e.g., retinal pigment epithelial cells), and precursor cells thereof (tissue precursor cells). Without particular limination concerning the degree of cell differentiation, the age of an animal from which cells are collected, or the like, both undifferentiated precursor cells (also including somatic stem cells) and terminally-differentiated mature cells can be similarly used as origins for somatic cells in the present invention. Examples of undifferentiated precursor cells include tissue stem cells (somatic stem cells) such as neural stem cells, hematopoietic stem cells, mesenchymal stem cells, and dental pulp stem cells.

In the present invention, individual mammals from which somatic cells are collected are not particularly limited but are preferably humans.

### Method for screening iPS cells

The above-established iPS cells are subjected to detection of the expression of miRNA in at least one imprinted region or a gene to be expressed from a maternally derived chromosome from among genes located in such at least one imprinting region, or, DNA methylation in a region controling expression of the gene locatedn in an imprinted region. Thus, iPS cells having unlimitedly high differentiation potency and being capable of germline transmission can be selected. In the present invention, the term "imprinted region" refers to a region encoding a gene that is selectively expressed from either maternally- or paternally-derived chromosome, wherein imprinted region is the Dlk1-Dio3 region.

The term "miRNA" as used herein refers to "pri-miRNA", "pre-miRNA" and "mature-miRNA", which concerns regulation of gene expression via inhibition of translation from mRNA to protein or mRNA degradation. The "pri-miRNA" is single strand RNA which transcribed from DNA and has a hairpin loop structure containing miRNA and its complementary strand. The "pre-miRNA" is produced from pri-miRNA partially cleaving by an intranuclear enzyme called Drosha. The "mature-miRNA" is single strand RNA (20-25 nucleotides) which is produced from pre-miRNA cleaveing by Dicer outside the nucleus. Therefore, miRNA to be detected in the present invention is not limited to any of these forms including pri-miRNA, pre-miRNA, and mature-miRNA.

miRNA in the present invention is miRNA transcribed from chromosome 12 in the case of mice and from chromosome 14 in the case of humans and is miRNA located in Dlk1-Dio3 region. In the case of mice, the pri-miRNA and mature-miRNA are respectively shown in Table 1 and Table 2. In the case of humans, the pri-miRNA and mature-miRNA are respectively shown in Table 3 and Table 4.

Examples of a method for detecting the above miRNA include, but are not particularly limited to, Northern blotting, hybridization such as *in situ* hybridization, an RNase protection assay, a PCR method, a real-time PCR method, and a microarray method.

A preferable detection method involves: the use of hybridization of either miRNA, which is/includes pri-miRNA and/or mature miRNA such as those listed in Tables 1 and 3 or Tables 2 and 4 (see below), or a gene such as that listed in Table 5 (see below), with a nucleic acid, which is capable of hybridizing with the miRNA or the gene, as a probe; or the use of a PCR method with primers, which are capable of amplifying a sequence of DNA encoding the miRNA or a sequence of the gene. According to the present invention, the miRNA or the gene is located in the Dlk1-Dio3 region, of an induced pluripotent stem cell. Preferably, the gene is MEG3 or MEG8.

Examples of the probe or primer nucleic acid include the whole or partial sequences of the RNA listed in Tables 1, 2, 3, and 4 or cDNA encoding the RNA, or the whole or partial sequences of the genes listed in Table 5 or cDNA thereof, or sequences complementary to said whole or partial sequences. The size of the probe is generally at least 15 nucleotides, preferably at least 20 nucleotides, for example 20-30 nucleotides, 30-70 nucleotides, 70-100 nucleotide or more, etc. The size of the primer is generally 17-30 or more, preferably 20-25. The synthesis of the probe or primer can be conducted chemically using a commercially available automated nucleic acid synthesizer, for example.

The probe also may be an artificial nucleic acid, such as LNA (locked nucleic acid) (this is also referred to as bridged nucleic acid (BNA)) or PNA (peptide nucleic acid), serving as an alternate for RNA having a sequence complementary to the nucleotide sequence of miRNA.

LNA has a cross-linked structure in which position 2' and position 4' of RNA ribose are covalently bound via methylene groups (A.A. Koshkin et al., Tetrahedron, 54: 3607 (1998); S. Obika et al., Tetrahedron Lett., 39: 5401 (1998)). PNA lacks ribose, but has a structure containing amide and ethylene imine bonds in the backbone. PNA is as described in P.E. Nielsen et al., Science 254: 1497 (1991), P.E.Nielsen ed., Peptide Nucleic Acids: Protocols and Applications, 2nd ed. Horizon Bioscience (UK) (2004), for example. miRNA to be detected and an artificial nucleic acid probe hybridizable thereto such as LNA and PNA bind onto carriers on a microarray or the like, so that a large number of miRNAs can be detected and quantitatively determined simultaneously. The size of an artificial nucleic acid may range from about 10 mer to 25 mer.

If necessary, the probe as described above may be labeled. As a label, a fluorescent label (e.g., cyan, fluorescamine, rhodamine, and a derivative thereof, such as Cy3, Cy5, FITC, and TRITC) can be used.

The number of miRNA to be detected may be any number and is at least 1, at least 5, at least 10, at least 20, at least 30, at least 40 or at least 50. More preferably the number of such miRNA is 36.

**Table 1 Pri-miRNA of mouse Dlk1-Dio3 region**

| ID | Accession | Sequence | SEQ ID NO: |
|---|---|---|---|
| mmu-mir-770 | MI0004203 | | 1 |
| mmu-mir-673 | MI0004601 | | 2 |
| mmu-mir-493 | MI0005514 | | 3 |
| mmu-mir-337 | MI0000615 | | 4 |
| mmu-mir-540 | MI0003518 | | 5 |
| mmu-mir-665 | MI0004171 | | 6 |
| mmu-mir-431 | MI0001524 | | 7 |
| mmu-mir-433 | MI0001525 | | 8 |
| | | | |
| mmu-mir-127 | MI0000154 | | 9 |
| mmu-mir-434 | MI0001526 | | 10 |
| mmu-mir-432 | MI0012528 | | 11 |
| mmu-mir-136 | MI0000162 | | 12 |
| mmu-miR -341 | MI0000625 | | 13 |
| mmu-mir-1188 | MI0006290 | | 14 |
| mmu-mir-370 | MI0001165 | | 15 |
| mmu-mir-882 | MI0005475 | | 16 |
| mmu-mir-379 | MI0000796 | | 17 |
| mmu-mir-411 | MI0001163 | | 18 |
| mmu-mir-299 | MI0000399 | | 19 |
| | | | |
| mmu-mir-380 | MI0000797 | | 20 |
| mmu-mir-1197 | MI0006305 | | 21 |
| mmu-mir-323 | MI0000592 | | 22 |
| mmu-mir-758 | MI0004129 | | 23 |
| mmu-mir-329 | MI0000605 | | 24 |
| mmu-mir-494 | MI0003532 | | 25 |
| mmu-mir-679 | MI0004638 | | 26 |
| mmu-mir-1193 | MI0006298 | | 27 |
| mmu-mir-666 | MI0004553 | | 28 |
| mmu-mir-543 | MI0003519 | | 29 |
| mmu-mir-495 | MI0004639 | | 30 |
| mmu-mir-667 | MI0004196 | | 31 |
| mmu-mir-376c | MI0003533 | | 32 |
| mmu-mir-654 | MI0005520 | | 33 |
| mmu-mir-376b | MI0001162 | | 34 |
| mmu-mir-376a | MI0000793 | | 35 |
| mmu-mir-300 | MI0000400 | | 36 |
| mmu-mir-381 | MI0000798 | | 37 |
| mmu-mir-487b | MI0003534 | | 38 |
| mmu-mir-539 | MI0003520 | | 39 |
| mmu-mir-544 | MI0005555 | | 40 |
| mmu-mir-382 | MI0000799 | | 41 |
| mmu-mir-134 | MI0000160 | | 42 |
| mmu-mir-668 | MI0004134 | | 43 |
| mmu-mir-485 | MI0003492 | | 44 |
| mmu-mir-453 | MI0005497 | | 45 |
| mmu-mir-154 | MI0000176 | | 46 |
| mmu-mir-496 | MI0004589 | | 47 |
| mmu-mir-377 | MI0000794 | | 48 |
| mmu-mir-541 | MI0003521 | | 49 |
| mmu-mir-409 | MI0001160 | | 50 |
| mmu-mir-412 | MI0001164 | | 51 |
| mmu-mir-369 | MI0003535 | | 52 |
| mmu-mir-410 | MI0001161 | | 53 |

**Table 2 Mature-miRNA of mouse Dlk1-Dio3 region**

| ID | Accession | Sequence | SEQ ID NO: |
|---|---|---|---|
| mmu-miR-770-3p | MIMAT0003891 | cgugggccugacguggagcugg | 54 |
| mmu-miR-770-5p | MIMAT0004822 | agcaccacgugucugggccacg | 55 |
| mmu-miR-673-3p | MIMAT0004824 | uccggggcugaguucugugcacc | 56 |
| mmu-miR-673-5p | MIMAT0003739 | cucacagcucugguccuuggag | 57 |
| mmu-miR-493 | MIMAT0004888 | ugaagguccuacugugugccagg | 58 |
| mmu-miR-337-3p | MIMAT0000578 | uucagcuccuauaugaugccu | 59 |
| mmu-miR-337-5p | MIMAT0004644 | gaacggcgucaugcaggaguu | 60 |
| mmu-miR-540-3p | MIMAT0003167 | aggucagaggucgauccugg | 61 |
| mmu-miR-540-5p | MIMAT0004786 | caagggucacccucugacucugu | 62 |
| mmu-miR-665 | MIMAT0003733 | accaggaggcugaggucccu | 63 |
| mmu-miR-431 | MIMAT0001418 | ugucuugcaggccgucaugca | 64 |
| mmu-miR-431* | MIMAT0004753 | caggucgucuugcagggcuucu | 65 |
| mmu-miR-433 | MIMAT0001420 | aucaugaugggcuccucggugu | 66 |
| mmu-miR-433* | MIMAT0001419 | uacggugagccugucauuauuc | 67 |
| mmu-miR-127 | MIMAT0000139 | ucggauccgucugagcuuggcu | 68 |
| mmu-miR-127* | MIMAT0004530 | cugaagcucagagggcucugau | 69 |
| mmu-miR-434-3p | MIMAT0001422 | uuugaaccaucacucgacuccu | 70 |
| mmu-miR-434-5p | MIMAT0001421 | gcucgacucaugguuugaacca | 71 |
| mmu-miR-432 | MIMAT0012771 | ucuuggaguagaucagugggcag | 72 |
| mmu-miR-136 | MIMAT0000148 | acuccauuuguuuugaugaugg | 73 |
| mmu-miR-136* | MIMAT0004532 | aucaucgucucaaaugagucuu | 74 |
| mmu-miR-341 | MIMAT0000588 | ucggucgaucggucggucggu | 75 |
| mmu-miR-1188 | MIMAT0005843 | uggugugagguugggccagga | 76 |
| mmu-miR-370 | MIMAT0001095 | gccugcugggguggaaccuggu | 77 |
| mmu-miR-882 | MIMAT0004847 | aggagagaguuagcgcauuagu | 78 |
| mmu-miR-379 | MIMAT0000743 | ugguagacuauggaacguagg | 79 |
| mmu-miR-411 | MIMAT0004747 | uaguagaccguauagcguacg | 80 |
| mmu-miR-411* | MIMAT0001093 | uauguaacacgguccacuaacc | 81 |
| mmu-miR-299 | MIMAT0004577 | uaugugggacgguaaaccgcuu | 82 |
| mmu-miR-299* | MIMAT0000377 | ugguuuaccgucccacauacau | 83 |
| mmu-miR-380-3p | MIMAT0000745 | uauguaguaugguccacaucuu | 84 |
| mmu-miR-380-5p | MIMAT0000744 | augguugaccauagaacaugcg | 85 |
| mmu-miR-1197 | MIMAT0005858 | uaggacacauggucuacuucu | 86 |
| mmu-miR-323-3p | MIMAT0000551 | cacauuacacggucgaccucu | 87 |
| mmu-miR-323-5p | MIMAT0004638 | aggugguccguggcgcguucgc | 88 |
| mmu-miR-758 | MIMAT0003889 | uuugugaccugguccacua | 89 |
| mmu-miR-329 | MIMAT0000567 | aacacacccagcuaaccuuuuu | 90 |
| mmu-miR-494 | MIMAT0003182 | ugaaacauacacgggaaaccuc | 91 |
| mmu-miR-679 | MIMAT0003455 | ggacugugaggugacucuuggu | 92 |
| mmu-miR-1193 | MIMAT0005851 | uaggucacccguuuuacuauc | 93 |
| mmu-miR-666-3p | MIMAT0004823 | ggcugcagcgugaucgccugcu | 94 |
| mmu-miR-666-5p | MIMAT0003737 | agcgggcacagcugugagagcc | 95 |
| mmu-miR-543 | MIMAT0003168 | aaacauucgcggugcacuucuu | 96 |
| mmu-miR-495 | MIMAT0003456 | aaacaaacauggugcacuucuu | 97 |
| mmu-miR-667 | MIMAT0003734 | ugacaccugccacccagcccaag | 98 |
| mmu-miR-376c | MIMAT0003183 | aacauagaggaaauuucacgu | 99 |
| mmu-miR-376c* | MIMAT0005295 | guggauauuccuucuauguuua | 100 |
| mmu-miR-654-3p | MIMAT0004898 | uaugucugcugaccaucaccuu | 101 |
| mmu-miR-654-5p | MIMAT0004897 | ugguaagcugcagaacaugugu | 102 |
| mmu-miR-376b | MIMAT0001092 | aucauagaggaacauccacuu | 103 |
| mmu-miR-376b* | MIMAT0003388 | guggauauuccuucuaugguua | 104 |
| mmu-miR-376a | MIMAT0000740 | aucguagaggaaaauccacgu | 105 |
| mmu-miR-376a* | MIMAT0003387 | gguagauucuccuucuaugagu | 106 |
| mmu-miR-300 | MIMAT0000378 | uaugcaagggcaagcucucuuc | 107 |
| mmu-miR-300* | MIMAT0004578 | uugaagagagguuauccuuugu | 108 |
| mmu-miR-381 | MIMAT0000746 | uauacaagggcaagcucucugu | 109 |
| mmu-miR-487b | MIMAT0003184 | aaucguacagggucauccacuu | 110 |
| mmu-miR-539 | MIMAT0003169 | ggagaaauuauccuuggugugu | 111 |
| mmu-miR-544 | MIMAT0004941 | auucugcauuuuuagcaagcuc | 112 |
| mmu-miR-382 | MIMAT0000747 | gaaguuguucgugguggauucg | 113 |
| mmu-miR-382* | MIMAT0004691 | ucauucacggacaacacuuuuu | 114 |
| mmu-miR-134 | MIMAT0000146 | ugugacugguugaccagagggg | 115 |
| mmu-miR-668 | MIMAT0003732 | ugucacucggcucggcccacuacc | 116 |
| mmu-miR-485 | MIMAT0003128 | agaggcuggccgugaugaauuc | 117 |
| mmu-miR-485* | MIMAT0003129 | agucauacacggcucuccucuc | 118 |
| mmu-miR-453 | MIMAT0004870 | agguugccucauagugagcuugca | 119 |
| mmu-miR-154 | MIMAT0000164 | uagguuauccguguugccuucg | 120 |
| mmu-miR-154* | MIMAT0004537 | aaucauacacgguugaccuauu | 121 |
| mmu-miR-496 | MIMAT0003738 | ugaguauuacauggccaaucuc | 122 |
| mmu-miR-377 | MIMAT0000741 | aucacacaaaggcaacuuuugu | 123 |
| mmu-miR-541 | MIMAT0003170 | aagggauucugauguuggucacacu | 124 |
| mmu-miR-409-3p | MIMAT0001090 | gaauguugcucggugaaccccu | 125 |
| mmu-miR-409-5p | MIMAT0004746 | agguuacccgagcaacuuugcau | 126 |
| mmu-miR-412 | MIMAT0001094 | uucaccugguccacuagccg | 127 |
| mmu-miR-369-3p | MIMAT0003186 | aauaauacaugguugaucuuu | 128 |
| mmu-miR-369-5p | MIMAT0003185 | agaucgaccguguuauauucgc | 129 |
| rnmu-miR-410 | MIMAT0001091 | aauauaacacagauggccugu | 130 |

**Table 3 Pri-miRNA of human Dlk1-Dio3 region**

| ID | Accession | Sequence | SEQ ID NO: |
|---|---|---|---|
| hsa-mir-770 | MI0005118 | | 131 |
| | | | |
| hsa-mir-493 | MI0003132 | | 132 |
| hsa-mir-337 | MI0000806 | | 133 |
| hsa-mir-665 | MI0005563 | | 134 |
| hsa-mir-431 | MI0001721 | | 135 |
| hsa-mir-433 | MI0001723 | | 136 |
| hsa-mir-127 | MI0000472 | | 137 |
| hsa-mir-432 | MI0003133 | | 138 |
| hsa-mir-136 | MI0000475 | | 139 |
| hsa-mir-370 | MI0000778 | | 140 |
| hsa-mir-379 | MI0000787 | | 141 |
| hsa-mir-411 | MI0003675 | | 142 |
| | | | |
| hsa-mir-299 | MI0000744 | | 143 |
| hsa-mir-380 | MI0000788 | | 144 |
| hsa-mir-1197 | MI0006656 | | 145 |
| hsa-mir-323 | MI0000807 | | 146 |
| hsa-mir-758 | MI0003757 | | 147 |
| hsa-mir-329-1 | MI0001725 | | 148 |
| hsa-mir-329-2 | MI0001726 | | 149 |
| hsa-mir-494 | MI0003134 | | 150 |
| hsa-mir-543 | MI0005565 | | 151 |
| hsa-mir-495 | MI0003135 | | 152 |
| hsa-mir-376c | MI0000776 | | 153 |
| hsa-mir-376a-2 | MI0003529 | | 154 |
| | | | |
| hsa-mir-654 | MI0003676 | | 155 |
| hsa-mir-376b | MI0002466 | | 156 |
| hsa-mir-376a-1 | MI0000784 | | 157 |
| hsa-mir-300 | MI0005525 | | 158 |
| hsa-mir-1185-1 | MI0003844 | | 159 |
| hsa-mir-1185-2 | MI0003821 | | 160 |
| hsa-mir-381 | MI0000789 | | 161 |
| hsa-mir-487b | MI0003530 | | 162 |
| hsa-mir-539 | MI0003514 | | 163 |
| hsa-mir-889 | MI0005540 | | 164 |
| hsa-mir-544 | MI0003515 | | 165 |
| hsa-mir-655 | MI0003677 | | 166 |
| | | | |
| hsa-mir-487a | MI0002471 | | 167 |
| hsa-mir-382 | MI0000790 | | 168 |
| hsa-mir-134 | MI0000474 | | 169 |
| hsa-mir-668 | MI0003761 | | 170 |
| hsa-mir-485 | MI0002469 | | 171 |
| hsa-mir-453 | MI0001727 | | 172 |
| hsa-mir-154 | MI0000480 | | 173 |
| hsa-mir-496 | MI0003136 | | 174 |
| hsa-mir-377 | MI0000785 | | 175 |
| hsa-mir-541 | MI0005539 | | 176 |
| hsa-mir-409 | MI0001735 | | 177 |
| hsa-mir-412 | MI0002464 | | 178 |
| hsa-mir-369 | MI0000777 | | 179 |
| hsa-mir-410 | MI0002465 | | 180 |
| hsa-mir-656 | MI0003678 | | 181 |

**Table 4 Mature-miRNA of human Dlk1-Dio3 region**

| ID | Accession | Sequence | SEQ ID NO: |
|---|---|---|---|
| hsa-miR-770-5p | MIMAT0003948 | uccaguaccacgugucagggcca | 182 |
| hsa-miR-493 | MIMAT0003161 | ugaaggucuacugugugccagg | 183 |
| hsa-miR-493* | MIMAT0002813 | uuguacaugguaggcuuucauu | 184 |
| hsa-miR-337-5p | MIMAT0004695 | gaacggcuucauacaggaguu | 185 |
| hsa-miR-337-3p | MIMAT0000754 | cuccuauaugaugccuuucuuc | 186 |
| hsa-miR-665 | MIMAT0004952 | accaggaggcugaggccccu | 187 |
| hsa-miR-431 | MIMAT0001625 | ugucuugcaggccgucaugca | 188 |
| hsa-miR-431* | MIMAT0004757 | caggucgucuugcagggcuucu | 189 |
| hsa-miR-433 | MIMAT0001627 | aucaugaugggcuccucggugu | 190 |
| hsa-miR-127-5p | MIMAT0004604 | cugaagcucagagggcucugau | 191 |
| hsa-miR-127-3p | MIMAT0000446 | ucggauccgucugagcuuggcu | 192 |
| hsa-miR-432 | MIMAT0002814 | ucuuggaguaggucauugggugg | 193 |
| hsa-miR-432* | MIMAT0002815 | cuggauggcuccuccaugucu | 194 |
| hsa-miR-136 | MIMAT0000448 | acuccauuuguuuugaugaugga | 195 |
| hsa-miR-136* | MIMAT0004606 | caucaucgucucaaaugagucu | 196 |
| hsa-miR-370 | MIMAT0000722 | gccugcugggguggaaccuggu | 197 |
| hsa-miR-379 | MIMAT0000733 | ugguagacuauggaacguagg | 198 |
| hsa-miR-379* | MIMAT0004690 | uauguaacaugguccacuaacu | 199 |
| hsa-miR-411 | MIMAT0003329 | uaguagaccguauagcguacg | 200 |
| hsa-miR-411* | MIMAT0004813 | uauguaacacgguccacuaacc | 201 |
| hsa-miR-299-5p | MIMAT0002890 | ugguuuaccgucccacauacau | 202 |
| hsa-miR-299-3p | MIMAT0000687 | uaugugggaugguaaaccgcuu | 203 |
| hsa-miR-380 | MIMAT0000735 | uauguaauaugguccacaucuu | 204 |
| hsa-miR-380* | MIMAT0000734 | ugguugaccauagaacaugcgc | 205 |
| hsa-miR-1197 | MIMAT0005955 | uaggacacauggucuacuucu | 206 |
| hsa-miR-323-5p | MIMAT0004696 | aggugguccguggcgcguucgc | 207 |
| hsa-miR-323-3p | MIMAT0000755 | cacauuacacggucgaccucu | 208 |
| hsa-miR-758 | MIMAT0003879 | uuugugaccugguccacuaacc | 209 |
| hsa-miR-329 | MIMAT0001629 | aacacaccugguuaaccucuuu | 210 |
| hsa-miR-494 | MIMAT0002816 | ugaaacauacacgggaaaccuc | 211 |
| hsa-miR-543 | MIMAT0004954 | aaacauucgcggugcacuucuu | 212 |
| hsa-miR-495 | MIMAT0002817 | aaacaaacauggugcacuucuu | 213 |
| hsa-miR-376c | MIMAT0000720 | aacauagaggaaauuccacgu | 214 |
| hsa-miR-376a | MIMAT0000729 | aucauagaggaaaauccacgu | 215 |
| hsa-miR-654-5p | MIMAT0003330 | uggugggccgcagaacaugugc | 216 |
| hsa-miR-654-3p | MIMAT0004814 | uaugucugcugaccaucaccuu | 217 |
| hsa-miR-376b | MIMAT0002172 | aucauagaggaaaauccauguu | 218 |
| hsa-miR-376a | MIMAT0000729 | aucauagaggaaaauccacgu | 219 |
| hsa-miR-376a* | MIMAT0003386 | guagauucuccuucuaugagua | 220 |
| hsa-miR-300 | MIMAT0004903 | uauacaagggcagacucucucu | 221 |
| hsa-miR-1185 | MIMAT0005798 | agaggauacccuuuguauguu | 222 |
| hsa-miR-381 | MIMAT0000736 | uauacaagggcaagcucucugu | 223 |
| hsa-miR-487b | MIMAT0003180 | aaucguacagggucauccacuu | 224 |
| hsa-miR-539 | MIMAT0003163 | ggagaaauuauccuuggugugu | 225 |
| hsa-miR-889 | MIMAT0004921 | uuaauaucggacaaccauugu | 226 |
| hsa-miR-544 | MIMAT0003164 | auucugcauuuuuagcaaguuc | 227 |
| hsa-miR-655 | MIMAT0003331 | auaauacaugguuaaccucuuu | 228 |
| hsa-miR-487a | MIMAT0002178 | aaucauacagggacauccaguu | 229 |
| hsa-miR-382 | MIMAT0000737 | gaaguuguucgugguggauucg | 230 |
| hsa-miR-134 | MIMAT0000447 | ugugacugguugaccagagggg | 231 |
| hsa-miR-668 | MIMAT0003881 | ugucacucggcucggcccacuac | 232 |
| hsa-miR-485-5p | MIMAT0002175 | agaggcuggccgugaugaauuc | 233 |
| hsa-miR-485-3p | MIMAT0002176 | gucauacacggcucuccucucu | 234 |
| hsa-miR-453 | MIMAT0001630 | agguuguccguggugaguucgca | 235 |
| hsa-miR-154 | MIMAT0000452 | uagguuauccguguugccuucg | 236 |
| hsa-miR-154* | MIMAT0000453 | aaucauacacgguugaccuauu | 237 |
| hsa-miR-496 | MIMAT0002818 | ugaguauuacauggccaaucuc | 238 |
| hsa-miR-377 | MIMAT0000730 | aucacacaaaggcaacuuuugu | 239 |
| hsa-miR-377* | MIMAT0004689 | agagguugcccuuggugaauuc | 240 |
| hsa-miR-541 | MIMAT0004920 | uggugggcacagaaucuggacu | 241 |
| hsa-miR-541* | MIMAT0004919 | aaaggauucugcugucggucccacu | 242 |
| hsa-miR-409-5p | MIMAT0001638 | agguuacccgagcaacuuugcau | 243 |
| hsa-miR-409-3p | MIMAT0001639 | gaauguugcucggugaaccccu | 244 |
| hsa-miR-412 | MIMAT0002170 | acuucaccugguccacuagccgu | 245 |
| hsa-miR-369-5p | MIMAT0001621 | agaucgaccguguuauauucgc | 246 |
| hsa-miR-369-3p | MIMAT0000721 | aauaauacaugguugaucuuu | 247 |
| hsa-miR-410 | MIMAT0002171 | aauauaacacagauggccugu | 248 |
| hsa-miR-656 | MIMAT0003332 | aauauuauacagucaaccucu | 249 |

In the present invention, genes located in imprinted region are genes located in the Dlk1-Dio3 region. Examples of such genes include Dlk1, Gtl2/Meg3, Rtl1, Rtl1 as, Meg8/Rian, Meg9/Mirg, and Dio3. The genes analysed in the present invention are imprinting genes that are expressed from only a maternally derived chromosome, which are shown in Table 5.

Examples of a method for detecting the expression of the above genes include, but are not particularly limited to, Northern blotting, Southern blotting, hybridization such as Northern hybridization, Southern hybridization, and *in situ* hybridization, RNase protection assay, a PCR method, quantitative PCR, a real-time PCR method, and a microarray method.

Detection can be performed by microarray method containing following steps of (i) extracting total RNA containing mRNA from a biological sample, (ii) obtaining mRNA using a poly T column, (iii) synthesizing cDNA by a reverse transcription reaction, (iv) amplifying using a phage or a PCR cloning method, and then (v) performing hybridization with a probe consisting of about 20 mer-70 mer or a larger size complementary to the target DNA or by quantitative PCR using about 20 mer-30 mer primers, for example. As a label for hybridization or PCR, a fluorescent label can be used. As such a fluorescent label, cyan, fluorescamine, rhodamine, or a derivative thereof such as Cy3, Cy5, FITC, and TRITC can be used.

The number of a gene to be detected may be any number and is at least 1, at least 2, or at least 3. More preferably the number of such gene is 4.

**Table 5 Maternally-derived genomic imprinting genes of Dlk1-Dio3 region**

| Gene name | Accession NO | |
|---|---|---|
| | Mouse | Human |
| Gtl2/MEG3 | NR_003633 (SEQ ID No:270) | NR_002766 (SEQ ID NO:274) |
| Rtl1 as/anti-Peg11 | NR_002848 (SEQ ID NO:271) | - |
| Rian/MEG8 | NR_028261 (SEQ ID NO:272) | NR_024149 (SEQ ID NO:275) |
| Mirg/Meg9 | NR_028265 (SEQ ID NO:273) | - |

Upon screening iPS cells having unlimitedly high differentiation potency and being capable of germline transmission, a value detected by the above method for control cells which are iPS cells or embryonic stem cells (ES cells) known to perform germline transmission is designated as the reference value (positive reference value). Subject iPS cells for which the value is equivalent to or higher than the positive reference value may be selected as iPS cells capable of germline transmission.

Similarly, a value detected by the above method for control cells which are iPS cells or embryonic stem cells (ES cells) that are known not to perform germline transmission is designated as the reference gene (negative reference gene). Subject iPS cells for which the value is higher than the negative reference value may be selected as iPS cells capable of germline transmission.

Another embodiment involves preparing Table 6 in advance using a series of cells known to perform or known not to be able to perform germline transmission and then designating the reference value so that the values for each or both sensitivity and specificity shown in Table 6 are 0.9 or more, preferably 0.95 or more, and more preferably 0.99 or more. When a value detected for subject iPS cells by the above method is equivalent to or higher than the reference value, the subject iPS cells can be screened for as iPS cells capable of germline transmission. Particularly preferably, the values for both sensitivity and specificity are 1. Here, a result in which both sensitivity and specificity are 1 indicates that the reference value is an identical reference value that will have neither a false-positive result nor a false-negative result.

**Table 6**

| | Number of iPS cells capable of germline transmission | Number of iPS cells incapable of germline transmission |
|---|---|---|
| Number of cell lines for which the detected value was the same as or higher than the reference gene | A | C |
| Number of cell lines for which the detected value was lower than the reference gene | B | D |
| | Sensitivity = A/(A+B) | Specificity = D/(C+D) |

Furthermore, in the present disclosure method for screening iPS cells capable of germline transmission may also be performed by detecting methylation of DNA in region controling expression of the gene located in the Dlkl-Dio3 region. At this time, an example of a region to be detected is a region that is referred to as a CpG island, which is the region having a high content of sequence consisting of cytosine and guanine, located between the region encoding Dlk1 and the region encoding Gtl2/MEG3, wherein its DNA methylation state in a maternally derived chromosomeis different from that in a paternally derived chromosome. A preferable example of such region is an intergenic differentially methylated region (IG-DMR) or MEG3-DMR (Gtl2-DMR). Examples of the above IG-DMR and MEG3-DMR include, but are not particularly limited to, regions as described in Cytogenet Genome Res 113:223-229, (2006), Nat Genet. 40:237-42, (2008) or Nat Genet. 35:97-102. (2003). A more specific example of the above IG-DMR is, in the case of mice, a region with a length of 351 bp ranging from nucleotide 80479 to nucleotide 80829 in the AJ320506 sequence of NCBI.

Examples of a method for detecting DNA methylation include methods that involve cleaving a subject recognition sequence using a restriction enzyme and methods that involve hydrolyzing unmethylated cytosine using bisulfite.

The former methods use a methylation-sensitive or -insensitive restriction enzyme, which is based on the fact that if a nucleotide in a recognition sequence is methylated, the cleaving activity of the restriction enzyme is altered. The thus generated DNA fragment is subjected to electrophoresis and then the fragment length of interest is measured by Southern blotting or the like, so that a methylated site is detected. On the other hand, the latter methods include a method that involves performing bisulfite treatment, PCR, and then sequencing, a method that involves using methylation-specific oligonucleotide (MSO) microarrays, or methylation-specific PCR that involves causing PCR primers to recognize a difference between a sequence before bisulfite treatment and the sequence after bisulfite treatment and then determining the presence or the absence of methylated DNA based on the presence or the absence of PCR products. In addition to these methods, by chromosome immunoprecipitation using a DNA methylation-specific antibody, DNA-methylated regions can be detected from specific regions by extracting DNA sequences within DNA-methylated regions, performing PCR, and then performing sequencing.

Upon screening iPS cells having unlimitedly high differentiation potency and being capable of germline transmission, subject iPS cells in which the subject region in one chromosome is in a DNA-methylated state, but the same region in homologous chromosome is not in a DNA-methylated state as detected by the above method can be selected as iPS cells having unlimitedly high differentiation potency or capable of germline transmission. Here, the expression, "the subject region in one chromosome is in a DNA-methylated state, but the same region in homologous chromosome is not in a DNA-methylated state" refers to, for example, a state in which the detected methylated CpGs in the subject region account for 30% or more and 70% or less, preferably 40% or more and 60% or less, more preferably 45% or more and 55% or less, and particularly preferably 50% of all detected CpGs. In a more preferable embodiment, a paternally derived chromosome alone is methylated and the same region of the maternally derived chromosome in the same cell is not methylated. As a result, it is desirable to select iPS cells for which detected methylated CpGs account for 50% of all detected CpGs.

As an example of a method for detecting the percentage of methylated CpGs, in the case of uisng a restriction enzyme recognizing unmethylated DNA, the percentage accounted for methylated DNAs can be calculated by compareing the amount of unfragmented DNA with fragmented DNA determined by Southern blotting. Meanwhile, in the case of the bisulfite method, arbitrarily selected chromosomes are sequenced. Hence, the percentage can be calculated by repeatedly sequencing a template to which a PCR product has been cloned a plurality of times such as 2 or more times, preferably 5 or more times, and more preferably 10 or more times and then comparing the number of sequenced clones with the number of clones for which DNA methylation has been detected. When a pyro-sequencing method is employed, the percentage can also be directly determined by measuring amout of cytosine or thymine (the amount of cytosine means amount of methylated DNAs and the amount of thymine means amount of unmethylated DNAs). Also, in the case of a chromosome immunoprecipitation method using a DNA methylation-specific antibody, the amount of precipitated DNA of interest and the amount of DNA before precipitation are detected by PCR and then compared, so that the percentage accounted for by methylated DNAs can be detected.

### Kit for screening of iPS cells

The kit for screening iPS cells according to the present disclosure contains a reagent for miRNA measurement, a reagent for gene measurement, or a reagent for measuring DNA methylation for the above detection method.

Examples of the reagent for miRNA measurement are probe or primer nucleic acids, including the whole or partial sequences of the RNA listed in Tables 1, 2, 3, and 4 or cDNA encoding the RNA. The size of the probe is generally at least 15 nucleotides, preferably at least 20 nucleotides, for example 20-30 nucleotides, 30-70 nucleotides, 70-100 nucleotide or more, etc.

The reagent for miRNA measurement also may contain, as an alternative to RNA having a sequence complementary to the nucleotide sequence of an miRNA shown in any of Tables 1-4 above, an artificial nucleic acid such as LNA (locked nucleic acid; also LNA referred to as bridged nucleic acid (BNA)) or PNA (peptide nucleic acid) as a probe.

A reagent for gene measurement can contain nucleic acid probes of a size of about 20 mer-70 mer or more in size that are fragments of target DNA or mRNA of an imprinting gene described in Table 5 above or nucleic acids complementary to the fragments, or a primer set or primers of about 20 mer-30 mer in size derived from said fragments and nucleic acids complementary thereto.

The kit can also contain microarrays prepared by binding the above-described probes to carriers, such as glassor polymers.

A reagent for DNA methylation measurement contains a reagent and microarrays to be used for an MSO (methylation-specific oligonucleotide) microarray method for detection of methylation of cytosine nucleotides using a bisulfite reaction (Izuho Hatada, Experimental Medicine, Vol. 24, No. 8 (Extra Number), pp. 212-219 (2006), YODOSHA (Japan)). In the bisulfite method, a single-stranded DNA is treated with bisulfite (sodium sulfite), so as to convert cytosine to uracil, but methylated cytosine is not converted to uracil. In a methylation specific oligonucleotide (MSO) microarray method, methylation is detected using a bisulfite reaction. In this method, PCR is performed for DNA treated with bisulfite by selecting sequences (containing no CpG sequences) that remain unaltered regardless of methylation as primers. As a result, unmethylated cytosine is amplified as thymine and methylated cytosine is amplified as cytosine. Oligonucleotides complementary to sequences in which thymine has been altered from unmethylated cytosine (in the case of unmethylated cytosine) and oligonucleotides complementary to sequences in which cytosine has remained unaltered (in the case of methylated cytosine) are immobilized to carriers of microarrays. The thus amplified DNA is fluorescence-labeled and then hybridized to the microarrays. Methylation can be quantitatively determined based on the occurrence of hybridization. A kit for determining a DNA methylation state of IG-DMR and/or Gtl2/MEG3-DMR for screening of induced pluripotent stem cells can contain a methylation-sensitive restriction enzyme, or a bisulfite reagent, and nucleic acids for amplification of IG-DMR and/or Gtl2/MEG3-DMR.

Example of the methylation-sensitive restriction enzymes include, but are not limited to, AatII, AccII, BssHII, ClaI, CpoI, Eco52I, HaeII, MluI, NaeI, NotI, NsbI, PvuI, SacII, SalI, etc.

The kit for screening iPS cells can also contain a reagent for miRNA extraction, a reagent for gene extraction, or a reagent for chromosome extraction, for example. Also, a kit for diagnosis may contain means for discrimination analysis such as documents or instructions containing procedures for discrimination analysis, a program for implementing the procedures for discrimination analysis by a computer, the program list, a recording medium containing the program recorded therein, which is readable by the computer (e.g., flexible disk, optical disk, CD-ROM, CD-R, and CD-RW), and an apparatus or a system (e.g., computer) for implementation of discrimination analysis.

The present invention will be further described in detail by examples as follows, but the scope of the present invention is not limited by these examples.

### EXAMPLES

### Mouse ES and iPS cells

Mouse ES cells (RF8, Nanog ES, and Fbx(-/-)ES) shown in Table 7 were cultured and sample iPS cells were established and cultured by conventional methods (Takahashi K and Yamanaka S, Cell 126 (4), 663, 2006; Okita K, et al., Nature 448 (7151), 313, 2007; Nakagawa M, et al., Nat Biotechnol 26 (1), 101, 2008, Aoi, T. et al., Science 321, 699-702, 2008; and Okita K, et al., Science 322, 949, 2008). Also, Table 7 shows the results of studying the generation of chimeric mice from each cell and the presence or the absence of germline transmission according to conventional methods. Here, "origin" indicates somatic cells serving as origins, "MEF" indicates Mouse Embryonic Fibroblast, "TTF" indicates Tail-Tip Fibroblast, "Hep" indicates hepatocytes, and "Stomach" indicates gastric epithelial cells. Also regarding "Transgene," "O" indicates Oct3/4, "S" indicates Sox2, "M" indicates c-Myc, and "K" indicates Klf4. Furthermore, "no (plasmid OSMK)" indicates that iPS cells were prepared by a plasmid method and no transgene was incorporated into a chromosome.

**Table 7 List of cells**

| Clone name | Cell type | Origin | Transgene | Adult chimera | Germline |
|---|---|---|---|---|---|
| RF8 | ES | blastocyst | - | (Yes) | (Yes) |
| Nanog ES | | | - | (Yes) | (Yes) |
| Fbx(-/-)ES | | | - | (Yes) | (Yes) |
| 20D17 | iPS | | OSMK | Yes | Yes |
| 38C2 | | | OSMK | Yes | No |
| 38D2 | | MEF | OSMK | Yes | No |
| 178B2 | | | OSK | Yes | No |
| 178B5 | | | OSK | Yes | Yes |
| 212C5 | | | OSMK | Yes | No |
| 212C6 | | | OSMK | Yes | No |
| 335D1 | | TTF | OSK | Yes | No |
| 335D3 | | | OSK | Yes | No |
| 256H13 | | | OSK | Yes | No |
| 256H18 | | | OSK | Yes | No |
| 98A1 | | Hep | OSMK | Yes | No |
| 103C1 | | | OSMK | Yes | Yes |
| 99-1 | | Stomach | OSMK | Yes | Yes |
| 99-3 | | | OSMK | Yes | Yes |
| 492B4 | | MEF | no (plasmid OSMK) | Yes | Yes |
| 492B9 | | | no (plasmid OSMK) | Yes | No |
| Fbx iPS 10-6 | | MEF | 10 factors | No | N.D. |
| Fbx iPS 4-7 | | | OSMK | No | N.D. |
| Fbx iPS 4-3 | | TTF | OSMK | No | N.D. |
| Fbx iPS WT1 | | | OSMK | No | N.D. |
| SNL feeder | | | | | |
| MEF | | | | | |
| TTF | Soma | | | | |
| Hepatocyte | | | | | |
| Stomach | | | | | |

### Human ES and iPS cells

Human ES cells (KhES1, KhES3, H1 and H9) were cultured, and iPS cell samples were established and cultured by conventional methods (Suemori H, et al., Biochem Biophys Res Commun, 345, 926-32, 2006, Thomson JA, et al., 282, 1145-7, 1998, US2009/0047263 and WO2010/013359). These cells were listed in Table 8, wherein "HDF" indicates Human Embryonic Fibroblast.

**Table 8 List of cells**

| Clone name | Cell type | Origin | Transgene |
|---|---|---|---|
| KhES1 | | | - |
| KhES3 | ES | blastocyst | - |
| H1 | | | - |
| H9 | | | - |
| 201B2 | iPS | HDF | OSMK |
| 201B6 | | | OSMK |
| 201B7 | | | OSMK |
| 253G1 | | | OSK |
| 253G4 | | | OSK |
| TIG103-4F4 | | | OSMK |
| TIG107-4F1 | | | OSK |
| TIG107-3F | | | OSMK |
| TIG108-4F3 | | | OSMK |
| TIG109-4F1 | | | OSMK |
| TIG114-4F1 | | | OSMK |
| TIG118-4F1 | | | OSMK |
| TIG120-4F1 | | | OSMK |
| TIG121-4F4 | | | OSMK |
| 1375-4F1 | | | OSMK |
| 1377-4F1 | | | OSMK |
| 1392-4F2 | | | OSMK |
| 1488-4F1 | | | OSMK |
| 1503-4F1 | | | OSMK |
| 1687-4F2 | | | OSMK |
| DP31-4F1 | | dental pulp | OSMK |
| 225C7 | | fetal HDF | OSMK |
| 246G 1 | | BJ cell | OSMK |

### Confirmation of microRNA expression in mouse cells

Profiling of the expression of microRNA expressed in mouse cells shown in Table 7 was performed using microRNA microarrays (Agilent).

211 probes determined to be ineffective for all the 29 samples were removed from 672 miRNA array probes. Hierarchical clustering was performed for a total of 461 probes. The results are shown in Fig. 1. Group I miRNA not expressed in somatic cells but expressed in ES cells and iPS cells and Group II miRNA expressed in various manners among iPS cells were extracted. Group I is shown in Fig. 2A and Table 9 and Group II is shown in Fig. 2B and Table 10. When Group II miRNA was analyzed, all members were found to be contained in the miRNA cluster of chromosome 12.

Group I miRNA was expressed to an extent equivalent to that in the case of ES cells in the case of iPS cell clones contributing to the birth of chimeric mice, but in the case of 4 clones of Fbx iPS cells not contributing to the birth of chimeric mice, only low expression levels were detected, compared with the case of ES cells. Thus, it was suggested that Group I miRNA can be used as a marker for iPS cells contributing to the birth of chimeric mice.

Group II miRNA was expressed in all clones (20D17, 178B5, 492B4, and 103C1) for which germline transmission could be confirmed, excluding 2 clones (99-1 and 99-3) of gastric-epithelial-cell-derived iPS cells. Also, among iPS clones prepared from MEF, the expression of Group II miRNA was observed in 2 clones (38C2 and 38D2) for which no germline transmission could be confirmed, but Group II miRNA was never expressed or expressed at levels lower than that in the case of ES cells in iPS clones prepared from TTF. It was suggested by the results that examination of Group II miRNA as a marker for iPS cells that are very similar to ES cells in which germline transmission occurs is useful.

**Table 9 Group I mouse miRNA**

| ID | Accession | Sequence | SEQ ID NO: |
|---|---|---|---|
| mmu-miR-290-5p | MIMAT0000366 | acucaaacuaugggggcacuuu | 250 |
| mmu-miR-290-3p | MIMAT0004572 | aaagugccgccuaguuuuaagccc | 251 |
| mmu-miR-291a-5p | MIMAT0000367 | caucaaaguggaggcccucucu | 252 |
| mmu-miR-291a-3p | MIMAT0000368 | aaagugcuuccacuuugugugc | 253 |
| mmu-miR-292-5p | MIMAT0000369 | acucaaacugggggcucuuuug | 254 |
| mmu-miR-292-3p | MIMAT0000370 | aaagugccgccagguuuugagugu | 255 |
| mmu-miR-293 | MIMAT0000371 | agugccgcagaguuuguagugu | 256 |
| mmu-miR-293* | MIMAT0004573 | acucaaacugugugacauuuug | 257 |
| mmu-miR-294 | MIMAT0000372 | aaagugcuucccuuuugugugu | 258 |
| mmu-miR-294* | MIMAT0004574 | acucaaaauggaggcccuaucu | 259 |
| mmu-miR-295 | MIMAT0000373 | aaagugcuacuacuuuugagucu | 260 |
| mmu-miR-295* | MIMAT0004575 | acucaaauguggggcacacuuc | 261 |

**Table 10 Group II mouse miRNA**

| ID | Accession | Sequence | SEQ ID NO: |
|---|---|---|---|
| mmu-miR-337-3p | MIMAT0000578 | uucagcuccuauaugaugccu | 59 |
| mmu-miR-337-5p | MIMAT0004644 | gaacggcgucaugcaggaguu | 60 |
| mmu-miR-431 | MIMAT0001418 | ugucuugcaggccgucaugca | 64 |
| mmu-miR-127 | MIMAT0000139 | ucggauccgucugagcuuggcu | 68 |
| mmu-miR-434-3p | MIMAT0001422 | uuugaaccaucacucgacuccu | 70 |
| mmu-miR-434-5p | MIMAT0001421 | gcucgacucaugguuugaacca | 71 |
| mmu-miR-136 | MIMAT0000148 | acuccauuuguuuugaugaugg | 73 |
| mmu-miR-136* | MIMAT0004532 | aucaucgucucaaaugagucuu | 74 |
| mmu-miR-341 | MIMAT0000588 | ucggucgaucggucggucggu | 75 |
| mmu-miR-379 | MIMAT0000743 | ugguagacuauggaacguagg | 79 |
| mmu-miR-411 | MIMAT0004747 | uaguagaccguauagcguacg | 80 |
| mmu-miR-411* | MIMAT0001093 | uauguaacacgguccacuaacc | 81 |
| mmu-miR-299* | MIMAT0000377 | ugguuuaccgucccacauacau | 83 |
| mmu-miR-380-3p | MIMAT0000745 | uauguaguaugguccacaucuu | 84 |
| mmu-miR-323-3p | MIMAT0000551 | cacauuacacggucgaccucu | 87 |
| mmu-miR-329 | MIMAT0000567 | aacacacccagcuaaccuuuuu | 90 |
| mmu-miR-543 | MIMAT0003168 | aaacauucgcggugcacuucuu | 96 |
| mmu-miR-495 | MIMAT0003456 | aaacaaacauggugcacuucuu | 97 |
| mmu-miR-376c | MIMAT0003183 | aacauagaggaaauuucacgu | 99 |
| mmu-miR-376b | MIMAT0001092 | aucauagaggaacauccacuu | 103 |
| mmu-miR-376b* | MIMAT0003388 | guggauauuccuucuaugguua | 104 |
| mmu-miR-376a | MIMAT0000740 | aucguagaggaaaauccacgu | 105 |
| mmu-miR-300 | MIMAT0000378 | uaugcaagggcaagcucucuuc | 107 |
| mmu-miR-381 | MIMAT0000746 | uauacaagggcaagcucucugu | 109 |
| mmu-miR-487b | MIMAT0003184 | aaucguacagggucauccacuu | 110 |
| mmu-miR-382 | MIMAT0000747 | gaaguuguucgugguggauucg | 113 |
| mmu-miR-382* | MIMAT0004691 | ucauucacggacaacacuuuuu. | 114 |
| mmu-miR-154 | MIMAT0000164 | uagguuauccguguugccuucg | 120 |
| mmu-miR-154* | MIMAT0004537 | aaucauacacgguugaccuauu | 121 |
| mmu-miR-377 | MIMAT0000741 | aucacacaaaggcaacuuuugu | 123 |
| mmu-miR-541 | MIMAT0003170 | aagggauucugauguuggucacacu | 124 |
| mmu-miR-409-3p | MIMAT0001090 | gaauguugcucggugaaccccu | 125 |
| mmu-miR-409-5p | MIMAT0004746 | agguuacccgagcaacuuugcau | 126 |
| mmu-miR-369-3p | MIMAT0003186 | aauaauacaugguugaucuuu | 128 |
| mmu-miR-369-5p | MIMAT0003185 | agaucgaccguguuauauucgc | 129 |
| mmu-miR-410 | MIMAT0001091 | aauauaacacagauggccugu | 130 |

### Confirmation of microRNA expression in human cells

Profiling of the expression of microRNA expressed in cells shown in Table 8 was performed using Human miRNA microarray V3 (Agilent).

The results of several probes of Group III human miRNA not expressed in somatic cells but expressed in ES cells and iPS cells and Group IV human miRNA of Dlk1-Dio3 region were are shown in Fig. 6 and 7. The list of Group III is shown Table 11 and the list of Group IV is shown in Table 12.

A lot of Group IV human miRNA was expressed in ES cell clones (KhES1 and KhES3) and iPS cell clones (201B2, 201B7, TIG103-4F4, TIG114-4F1, TIG120-4F1, 1375-4F1, 1687-4F2 and DP31):

**Table 11 Group III human miRNA**

| ID | Accession | Sequence | SEQ ID NO: |
|---|---|---|---|
| hsa-miR-302a* | MIMAT0000683 | acuuaaacguggauguacuugcu | 262 |
| hsa-miR-367 | MIMAT0000719 | aauugcacuuuagcaaugguga | 263 |
| hsa-miR-302c | MIMAT0000717 | uaagugcuuccauguuucagugg | 264 |
| hsa-miR-302d | MIMAT0000718 | uaagugcuuccauguuugagugu | 265 |
| hsa-miR-302c* | MIMAT0000716 | uuuaacauggggguaccugcug | 266 |
| hsa-miR-302b* | MIMAT0000714 | acuuuaacauggaagugcuuuc | 267 |
| hsa-miR-302a | MIMAT0000684 | uaagugcuuccauguuuugguga | 268 |
| hsa-miR-302b | MIMAT0000715 | uaagugcuuccauguuuuaguag | 269 |

**Table 12 Group IV human miRNA**

| ID | Accession | Sequence | SEQ ID NO: |
|---|---|---|---|
| hsa-miR-369-3p | MIMAT0000721 | aauaauacaugguugaucuuu | 247 |
| hsa-miR-656 | MIMAT0003332 | aauauuauacagucaaccucu | 249 |
| hsa-miR-431* | MIMAT0004757 | caggucgucuugcagggcuucu | 189 |
| hsa-miR-433 | MIMAT0001627 | aucaugaugggcuccucggugu | 190 |
| hsa-miR-299-3p | MIMAT0000687 | uaugugggaugguaaaccgcuu | 203 |
| hsa-miR-136* | MIMAT0004606 | caucaucgucucaaaugagucu | 196 |
| hsa-miR-136 | MIMAT0000448 | acuccauuuguuuugaugaugga | 195 |
| hsa-miR-654-3p | MIMAT0004814 | uaugucugcugaccaucaccuu | 217 |
| hsa-miR-299-5p | MIMAT0002890 | ugguuuaccgucccacauacau | 202 |
| hsa-miR-493* | MIMAT0002813 | uuguacaugguaggcuuucauu | 184 |
| hsa-miR-382 | MIMAT0000737 | gaaguuguucgugguggauucg | 230 |
| hsa-miR-376a* | MIMAT0003386 | guagauucuccuucuaugagua | 220 |
| hsa-miR-409-3p | MIMAT0001639 | gaauguugcucggugaaccccu | 244 |
| hsa-miR-127-3p | MIMAT0000446 | ucggauccgucugagcuuggcu | 192 |
| hsa-miR-409-5p | MIMAT0001638 | agguuacccgagcaacuuugcau | 243 |
| hsa-miR-539 | MIMAT0003163 | ggagaaauuauccuuggugugu | 225 |
| hsa-miR-410 | MIMAT0002171 | aauauaacacagauggccugu | 248 |
| hsa-miR-495 | MIMAT0002817 | aaacaaacauggugcacuucuu | 213 |
| hsa-miR-379 | MIMAT0000733 | ugguagacuauggaacguagg | 198 |
| hsa-miR-377 | MIMAT0000730 | aucacacaaaggcaacuuuugu | 239 |
| hsa-miR-376a | MIMAT0000729 | aucauagaggaaaauccacgu | 219 |
| hsa-miR-381 | MIMAT0000736 | uauacaagggcaagcucucugu | 223 |
| hsa-miR-487b | MIMAT0003180 | aaucguacagggucauccacuu | 224 |
| hsa-miR-337-5p | MIMAT0004695 | gaacggcuucauacaggaguu | 185 |
| hsa-miR-411 | MIMAT0003329 | uaguagaccguauagcguacg | 200 |
| hsa-miR-411* | MIMAT0004813 | uauguaacacgguccacuaacc | 201 |
| hsa-miR-329 | MIMAT0001629 | aacacaccugguuaaccucuuu | 210 |
| hsa-miR-431 | MIMAT0001625 | ugucuugcaggccgucaugca | 188 |
| hsa-miR-323-3p | MIMAT0000755 | cacauuacacggucgaccucu | 208 |
| hsa-miR-758 | MIMAT0003879 | uuugugaccugguccacuaacc | 209 |
| hsa-miR-376b | MIMAT0002172 | aucauagaggaaaauccauguu | 218 |
| hsa-miR-154* | MIMAT0000453 | aaucauacacgguugaccuauu | 237 |
| hsa-miR-370 | MIMAT0000722 | gccugcugggguggaaccuggu | 197 |
| hsa-miR-432 | MIMAT0002814 | ucuuggaguaggucauugggugg | 193 |
| hsa-miR-154 | MIMAT0000452 | uagguuauccguguugccuucg | 236 |
| hsa-miR-337-3p | MIMAT0000754 | cuccuauaugaugccuuucuuc | 186 |
| hsa-miR-485-3p | MIMAT0002176 | gucauacacggcucuccucucu | 234 |
| hsa-miR-369-5p | MIMAT0001621 | agaucgaccguguuauauucgc | 246 |
| hsa-miR-377* | MIMAT0004689 | agagguugcccuuggugaauuc | 240 |
| hsa-miR-493 | MIMAT0003161 | ugaaggucuacugugugccagg | 183 |
| hsa-miR-485-5p | MIMAT0002175 | agaggcuggccgugaugaauuc | 233 |
| hsa-miR-494 | MIMAT0002816 | ugaaacauacacgggaaaccuc | 211 |
| hsa-miR-134 | MIMAT0000447 | ugugacugguugaccagagggg | 231 |
| hsa-miR-379* | MIMAT0004690 | uauguaacaugguccacuaacu | 199 |
| hsa-miR-380 | MIMAT0000735 | uauguaauaugguccacaucuu | 204 |
| hsa-miR-487a | MIMAT0002178 | aaucauacagggacauccaguu | 229 |
| hsa-miR-654-5p | MIMAT0003330 | uggugggccgcagaacaugugc | 216 |
| hsa-miR-668 | MIMAT0003 881 | ugucacucggcucggcccacuac | 232 |
| hsa-miR-376c | MIMAT0000720 | aacauagaggaaauuccacgu | 214 |
| hsa-miR-543 | MIMAT0004954 | aaacauucgcggugcacuucuu | 212 |

### Confirmation of mouse mRNA expression of Dlk1, Meg3/Gtl2, Meg8/Rian, Meg9/Mirg, and Dio3 gene

Expression of Dlk1, Meg3/Gtl2, Meg8/Rian, Meg9/Mirg, and Dio3 encoded by the same gene sites as in the case of the above Group II miRNA was examined using gene expression arrays (Agilent). The results are shown in Fig. 4. The Dlk1 gene and Dio3 gene that are expressed only in a paternally derived chromosome were expressed in almost the same manner among iPS cells clones. However, Meg3/Gtl2, Meg8/Rian, and Meg9/Mirg genes that are expressed only in a maternally derived chromosome were expressed in various manners among iPS cell clones and the distribution of the expression correlated with that for Group II miRNA above. Therefore, it was suggested that the genes that are expressed only in a maternally derived chromosome are useful as markers for iPS cells having functions equivalent to those of ES cells in which germline transmission occurs.

### Confirmation of human mRNA expression of MEG3 and MEG8 gene

Expression of MEG3 mRNA and MEG8 mRNA in ES cells and iPS cells was examined using Quantitative-PCR (qPCR) by Taqman probe whose assay ID of MEG3, MEG8 and GAPDH as internal standard were respectively Hs00292028_m1, Hs00419701_m1 and Hs03929097_g1 (Applied biosystems). The results are shown in Fig. 8. KhES1, 201B2, 201B7, TIG103-4F4, TIG114-4F1, TIG120-4F1, 1375-4F1, 1687-4F2 and DP31-4F1 were highly expressing these genes. Thus, these genes expression were correlated with the exression of miRNA located in human DLK1-DIO3 region shown in Figs. 6 and 7.

### Confirmation of DNA methylation of IG-DMR and MEG3-DMR

Methylation of IG-DMR (see Cytogenet Genome Res 113: 223-229, (2006)) was examined for germline-competent mouse iPS cells (178B5) prepared by introducing 3 genes (OSK) into MEF, ES cells (RF8) and and iPS cells (335D3) prepared by introducing 3 genes (OSK) into TTF for which no germline transmission had been confirmed. Specifically, DNA methylation of the CG sequence in a 351-bp portion ranging from nucleotide 80479 to nucleotide 80829 in the AJ320506 sequence (NCBI) was measured. DNA methylation was confirmed by treating DNA extracted from subject cells using a MethylEasy Xceed Rapid DNA Bisulphite Modification Kit (Human genetics) as a reagent for bisulfite treatment, amplifying IG-DMR by PCR, and then analyzing the cloned PCR products using a capillary sequencer. The experiment was conducted a plurality of times. One of the results is shown in Fig. 5. In the case of ES cells (RF8), 62% of 61 clones measured were methylated; and in the case of 178B5 iPS cells, 50% of 54 clones measured were methylated. This is inferred to be a state in which either a paternally-derived or a maternally-derived chromosome alone was methylated. Hence, it is considered that normal imprinting was carried out in these two cell lines. On the other hand, in the case of 335D3 iPS cells in which no germline transmission occurs, results indicating abnormal imprinting (e.g., when all CpG cytosines had been methylated) were obtained. Accordingly, it was suggested that iPS cells in which germline transmission occurs can be screened for by measuring IG-DMR methylation and then confirming if imprinting of the region is normal or not.

Similarly, the concentration of methylated cytosine in IG-DMR CG4 and MEG3-DMR CG7 shown in Fig. 9 was examind in human cells. The result of each clones (KhES1, DP31-4F1, KhES3, 201B7, H1 and 201B6) is shown in Fig. 10, wherein KhES1 and DP31-4F1 were exemplified as the high MEG3 expression clones, KhES3 and 201B7 as middle MEG3 expression clones and H1 and 201B6 as low MEG3 expression clones according to result of qPCR shown in Fig. 8. The degree of DNA methylation in IG-DMR CG4 and MEG3-DMR CG7 was inversely-correlating with the expression of MEG3 and MEG8 mRNA. For example, 65% cytosines in IG-DMR CG4 were methylated in IG-DMR of DP31-4F1 which was highly expressing MEG3 and MEG8 mRNA. On the contrary, 93% cytosine in IG-DMR CG4 were methylated in IG-DMR of 201B6 which less expressed MEG3 and MEG8 mRNA.

Meanwhile, it was examined whether undifferentiated cells expressing Oct-3/4 genes were include in the differentiated neural cells from each ES cells or iPS cells using SFEBq method. The said SFEBq method was performed with method comprising following steps of:
(i) the ES cells or iPS cells were cultured with medium containg Y27632;
(ii) for removal of feeder cells CTK dissociation solution (0.25% Trypsin, 1 mg/ml Collagenase and KSR 20%, and 1 mM CaCl2) was added to culture dish and transfer to gelatin coated dish;
(iii) the ES cells or iPS cells were dissociated with Accumax (TM);
(iv) the dissociated ES cells or iPS cells were transfer to LIPIDURE-COAT PLATE (NOF Corporation) and cultured with differentiation medium (DMEM/Ham's F12 containing 5% knockout serum replacement (KSR), 2 mM L-glutamine, non-essential amino acids, and 1 micro-M 2-mercaptoethanol (2-ME)) contained 10 micro-M Y27632, 2 micro-M Dorsomorphin (Sigma) and 10 micro-M SB431542 (Sigma) for 3 or 4 days; and
(v) Half media was changed with new differentiation medium without Y27632, Dorsomorphin and SB431542 and cultured for more 10 or 11 days.

After the differentiation to neural cells, clones of TIG108-4F3 (relative value of MEG3 and MEG8 mRNA expression shown in Fig. 8 are 0 and 0.00083) and TIG118-4F1 (relative value of MEG3 and MEG8 mRNA expression shown in Fig. 8 are 0.012 and 0.017) still included Oct3/4 positive cells when checking by flow cytometer. On the contrary, clones of KhES1, 201B7 (relative value of MEG3 and MEG8 mRNA expression shown in Fig. 8 are 0.61 and 0.64) and so on included no Oct3/4 positive cells.

These result showed that degree of DNA methylated in IG-DMR and MEG3-DMR and expression of MEG3 and/or MEG8 were able to be used as the marker of quality (e.g. pluripotency and ability for easy induction of differentiation) of iPS cells.
## Claims

1. A method of screening for an induced pluripotent stem cell(s) having unlimitedly high differentiation potency, comprising the following steps of:
(1) measuring the expression level of gene(s) or miRNA located in an imprinted region in a subject induced pluripotent stem cell(s); and,
(2) selecting the induced pluripotent stem cell(s) expressing the gene(s) or the miRNA at a higher level than that of a control cell(s),
wherein the miRNA located in an imprinted region is selected from the group consisting of the pri-miRNAs shown in Tables 1 and 3 and the mature-miRNAs shown in Tables 2 and 4, wherein the gene(s) located in an imprinted region is/are selected from the group consisting of Gtl2/MEG3, Rt11as/anti-Peg11, Rian/MEG8, and Mirg/Meg9, and wherein the control cell(s) is/are an iPS cell(s) or an embryonic stem cells (ES cells) known not to have unlimitedly high differentiation potency.

## Patentansprüche

1. Screening-Verfahren für eine induzierte pluripotente Stammzelle(n) mit unbegrenzt hoher Differenzierungspotenz, das die folgenden Schritte umfasst:
(1) Messen des Expressionsniveaus eines Gens bzw. von Genen oder miRNA, das bzw. die in einem geprägten Bereich in einer gegenständlichen induzierten pluripotenten Stammzelle(n) lokalisiert ist bzw. sind; und
(2) Auswählen der induzierten pluripotenten Stammzelle(n), die das Gen bzw. die Gene oder die miRNA auf einem höheren Niveau als dem einer Kontrollzelle bzw. von Kontrollzellen exprimiert bzw. exprimieren,
wobei die in einem geprägten Bereich lokalisierte miRNA aus der aus den pri-miRNAs gemäß Tabellen 1 und 3 und den mature-miRNAs gemäß Tabellen 2 und 4 bestehenden Gruppe ausgewählt ist, wobei das Gen bzw. die Gene, das bzw. die in einem geprägten Bereich lokalisiert ist bzw. sind, aus der aus Gt12/MEG3, Rt11as/anti-Peg11, Rian/MEG8 und Mirg/Meg9 bestehenden Gruppe ausgewählt ist bzw. sind und wobei es sich bei der bzw. den Kontrollzelle(n) um eine iPS-Zelle(n) oder eine embryonale Stammzelle(n) (ES-Zellen) handelt, die bekanntermaßen keine unbegrenzt hohe Differenzierungspotenz aufweist bzw. aufweisen.

## Revendications

1. Procédé de criblage d'une ou plusieurs cellules souches pluripotentes induites présentant une capacité de différentiation illimitée, comprenant les étapes suivantes:
(1) mesurer le niveau d'expression d'un ou plusieurs gènes ou d'un miARN situés dans une région soumise à empreinte dans une ou plusieurs cellules souches pluripotentes induites examinées ; et
(2) sélectionner la ou les cellules souches pluripotentes induites exprimant le ou les gènes ou le miARN à un niveau plus élevé que celui d'une ou plusieurs cellules témoins,
dans lequel le miARN situé dans une région soumise à empreinte est sélectionné dans le groupe constitué des pri-miARN indiqués dans les Tableaux 1 et 3 et des miARN matures indiqués dans les Tableaux 2 et 4, dans lequel le ou les gènes situés dans une région soumise à empreinte sont sélectionnés dans le groupe constitué de Gt12/MEG3, Rtl1as/anti-Peg11, Rian/MEG8 et Mirg/Meg9, et dans lequel la ou les cellules témoins sont une ou plusieurs cellules SPi ou cellules souches embryonnaires (cellules ES) dont on sait qu'elles ne présentent pas de capacité de différenciation illimitée.
